# EUROPEAN PATENT APPLICATION

(11) **EP 4 147 715 A1**
(43) Date of publication of application: **15.03.2023**
(21) Application number: 22182920.3
(22) Date of filing: 21.07.2017
(51) Int. Cl.: A61K 39/015, A61K 39/385, A61K 39/00

(54) **BIOFUSION PROTEINS AS ANTI-MALARIA VACCINES**

(30) Priority: 21.07.2016 EP 16180544
(62) Divisional of application: 17746213.2
(71) Applicant: VAC4all Pte. Ltd., Singapore 588177 (SG)
(72) Inventor: Druilhe, Pierre, 75015 PAris (FR)
(74) Representative: Ernest Gutmann - Yves Plasseraud S.A.S.

(57) **Abstract**

The invention relates to fusion proteins which comprise at least one antigenic amino acid sequence fused to a carrier heterologous protein sequence, wherein the antigenic sequence comprises an epitopic sequence of a Plasmodium protein and the carrier heterologous protein sequence is a sequence that is immunogenic in humans. The proteins are useful as anti-malaria vaccines.

## Description

The present invention relates to the protection against malaria, more particularly to a malaria vaccine which comprises a fusion protein.

### Background of the invention

The parasites responsible for malaria in human, including especially *Plasmodium falciparum,* exhibit different morphologies in the human host and express different antigens as a function of their localization in the organism of the infected host. The morphological and antigenic differences of these parasites during their life cycle in man enable at least four distinct stages of development. The very first stage of development of the parasite in human corresponds to the sporozoite form introduced into the blood of the host by bites of insect vectors of the parasite. The second stage corresponds to the passage of the parasite into the liver and to the infection of the hepatic cells in which the parasites develop to form the hepatic schizonts which, when they are mature (for example, in the case of *P. falciparum* on the 6th day after penetration of the sporozoites) release hepatic merozoites by bursting. The third stage is characterized by the infection of the blood erythrocytes by the asexual forms (blood stages schizonts) of the parasite releasing free merozoites when they mature; this erythrocytic stage of development corresponds to the pathogenic phase of the disease. The fourth stage corresponds to the formation of the forms with sexual potential (or gametocytes) which will become extracellular sexual forms or gametes in the mosquito.

There is no practical or effective vaccine that has been introduced into clinical practice.

Malaria sub-unit vaccines have so far shown a major deficiency in immunogenicity. The immune responses that are observed are at low titers and of short duration. A variety of approaches have been explored to address this deficiency, including a selection of adjuvants, expression in complex vectors such as virus-like particles (VLPs), adenovirus vectors, and setting up of complex prime-boost protocols. Even the RTS,S/AS01 vaccine (Mosquirix^{®}), which was engineered using genes from the repeat and T-cell epitope in the pre-erythrocytic circumsporozoite protein (CSP) of the Plasmodium falciparum malaria parasite and a viral envelope protein of the hepatitis B virus (HBsAg), with sophisticated adjuvants such as ASO1, suffers from very short duration of malaria-specific immune responses (whereas hepatitis B responses remain high for long).

Chemical conjugation of Plasmodium antigens to carrier proteins has also been disclosed. For instance a vaccine comprising Plasmodium protein Pfs25H conjugated to a detoxified form of Pseudomonas aeruginosa exoprotein A (repA) is under evaluation.

However chemical conjugation has several drawbacks. It is technically demanding, hardly reproducible, and quite expensive.

There is still a great need for vaccine products which show a good immunogenicity and protection against malaria, while being easy to reproduce and affordable.

### Summary of the invention

The invention offers the advantage of an improved immunogenicity, thanks to novel constructs consisting of Plasmodium polypeptides fused with a carrier protein such as CRM197. Rather than coupling chemically the Plasmodium molecule to the carrier protein, the inventors developed a novel approach in which the parasite protein is encoded together with the carrier protein as a fusion protein, making a "bio-conjugated fusion product", also designated further herein as "bio-fusion molecule", which can be produced recombinantly, e.g. in E.coli.

The invention more particularly provides a fusion protein which comprises at least one antigenic amino acid sequence fused at the N-term and/or C-term of a carrier heterologous protein sequence, wherein the antigenic sequence comprises an epitope sequence of a Plasmodium protein and the carrier heterologous protein sequence is a sequence that is immunogenic in humans.

The antigenic sequence may be an antigenic protein which is expressed by Plasmodium at erythrocytic stage, or an epitopic fragment thereof.

Alternatively, the antigenic sequence may be an antigenic protein which is expressed by Plasmodium at pre-erythrocytic stage, or an epitopic fragment thereof.

A further object of the invention is a nucleic acid construct encoding the fusion protein as defined herein.

Also provided are a vector comprising the nucleic acid construct in an expression cassette as well as a host cell wherein the vector has been inserted.

It is further described an *in vitro* method for preparing a fusion protein, which method comprises allowing the host cell to reproduce under conditions which induce expression of the nucleic acid construct, and collecting the fusion protein which is so expressed.

According to the invention, a vaccine is provided, which comprises said fusion protein or a combination of at least two fusion proteins, which respectively comprise at least two different antigenic amino acid sequences, or a nucleic acid construct encoding said fusion protein(s), with a physiologically acceptable vehicle.

Such vaccine is useful in vaccinating a human subject against malaria.

According to particular aspects, are described herein:
Item 1.A fusion protein which comprises at least one antigenic amino acid sequence fused at the N-term and/or C-term of a carrier heterologous protein sequence, wherein the antigenic sequence comprises an epitope sequence of a Plasmodium protein and the carrier heterologous protein sequence is a sequence that is immunogenic in humans.
Item 2. The fusion protein of item 1, wherein the carrier heterologous protein sequence is selected from the group consisting of a cross-reacting material (CRM) of diphtheria toxoid, diphtheria toxoid (D), a non-toxic mutant recombinant Pseudomonas aeruginosa exoprotein A (repA), meningococcal outer membrane protein complex (OMPC), tetanus toxoid (T), *H. influenza* protein D (hiD), and immunogenic fragments thereof, wherein the carrier heterologous protein sequence preferably is CRM197 or an immunogenic fragment thereof, which fragment is preferably selected from the group consisting of Fragment A, Transmembrane Domain T, Receptor Binding domain R of CRM197, amino acid sequence 1-190 of CRM197, and amino acid sequence 1-389 of CRM197.
Item 3. The fusion protein according to any of items 1 or 2, wherein the antigenic sequence is an antigenic protein which is expressed by Plasmodium at erythrocytic stage, or an epitopic fragment thereof.
Item 4. The fusion protein of item 3, wherein the antigenic sequence is selected from the group consisting of MSP3, LSA5 (also named PEBS or sub-region of 11-1), Glurp R0 and R2, SERP, P27 and P27A, P45, P90 and P77, P14, P181, MSP1-Block 2, MSP2, GBP 130, protein 332, protein 11-1, MSP4, or any epitopic fragment thereof, wherein the antigenic sequence preferably is MSP3 or an epitopic fragment thereof, which fragment preferably encompasses motifs a, b, c, d, e and/or f of the C-terminal region of a Plasmodium MSP3 protein chosen among MSP3-1, MSP3-2, MSP3-3, MSP3-4, MSP3-7, and MSP3-8, preferably MSP3-1.
Item 5. The fusion protein according to any of items 1 or 2, wherein the antigenic sequence is an antigenic protein which is expressed by Plasmodium at pre-erythrocytic stage, or an epitopic fragment thereof.
Item 6. The fusion protein according to item 5, wherein the antigenic sequence is selected from the group consisting of LSA3, LSA5 (also named PEBS or sub-region of 11-1 )" CS, Trap, and Salsa, and any epitopic fragment thereof.
Item 7. The fusion protein according to any of items 1 or 2, wherein the antigenic sequence is selected from the group consisting of Pf25, Pf45/48, Rh5, AMA1, MSP1-19, MSP1-42, MSP2, MSP4-5, Var CSA, and any epitopic fragment thereof.
Item 8. The fusion protein according to any of items 1-6 comprising MSP3, preferably MSP3-1, LSA3, LSA5, or an epitopic fragment thereof, as the antigenic sequence, fused to CRM197 or an immunogenic fragment thereof, as the carrier sequence.
Item 9. The fusion protein according to any of items 1-8, comprising two antigenic sequences which are either each located in C-term and N-term of the carrier protein sequence, or both located at the same terminus.
Item 10. The fusion protein according to any of items 1-8, wherein the at least one amino acid sequence is linked at the N-term and/or C-term of the carrier heterologous protein sequence by a peptide linker, wherein the peptide linker preferably is a sequence of 1 to 35 amino acids, preferably 5 to 20 amino acids.
Item 11. The fusion protein according to item 10, wherein the peptide linker is (Gly-Gly-Gly-Gly-Ser)n, (Gly)n or (EAAAK)ₙ, wherein n is 1 to 4, preferably 1 to 3.
Item 12. A nucleic acid construct encoding the fusion protein as defined in any of items 1 to 11.
Item 13. A vector comprising the nucleic acid construct of item 12 in an expression cassette.
Item 14. A host cell wherein the vector of item 13 has been inserted.
Item 15. An *in vitro* method for preparing a fusion protein as defined in any of items 1 to 11, which method comprises allowing the host cell of item 14 to reproduce under conditions which induce expression of the nucleic acid construct as defined in item 12, and collecting the fusion protein which is so expressed.
Item 16. A vaccine comprising the fusion protein as defined in any of items 1 to 11, or a combination of at least two fusion proteins as defined in any of items 1 to 11, which respectively comprise at least two different antigenic amino acid sequences, or a nucleic acid construct encoding said fusion protein(s), as defined in item 12, with a physiologically acceptable vehicle.
Item 17. The vaccine of item 16, for use in vaccinating a human subject against malaria.

### Legends to the figures

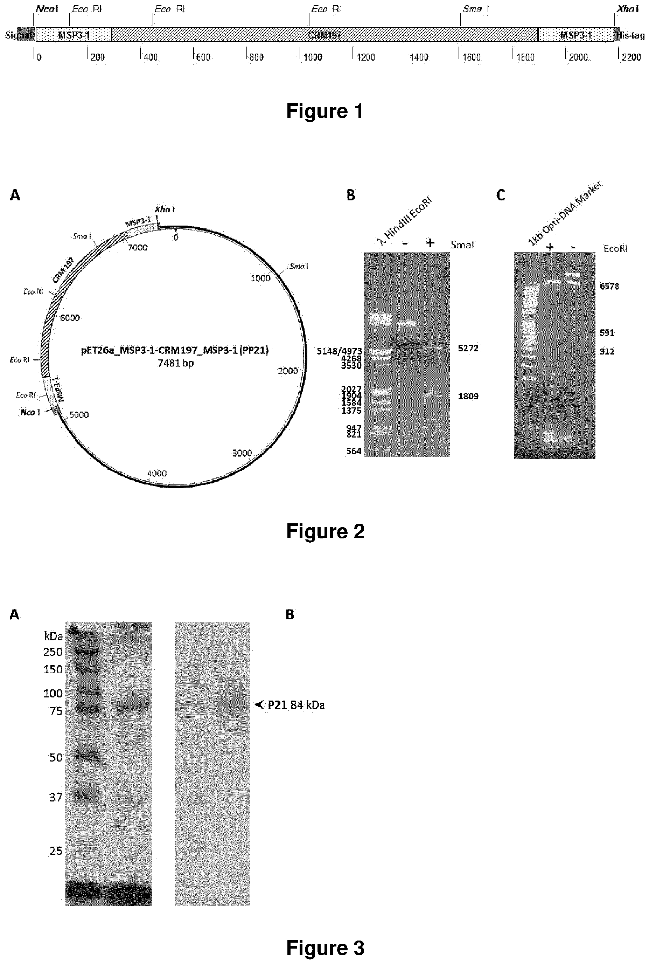

**Figure 1** is a diagramatic representation of the coding sequences for PP21 (also named "Bio-fusion MSP3-1). Scales in base pairs are shown below starting at the Nco I site.
**Figure 2** shows pET26a-PP21 plasmid map (A), mini-preparation analysis by restriction enzyme digestion with Sma I (B) or Eco R I (C).
**Figure 3****:** Recombinant protein PP21 purified by IMAC. (A) Analysis by electrophoresis under denaturing conditions and Coomassie blue staining. (B) Antigenicity assessed by Western blotting with murine anti-MSP3 immune sera at a 1:1000 dilution. The upper band observed is compatible with the theoretical molecular weight of the full length PP21 of 84 kDa. Another band at 37 kDa, less well recognized by the anti-MSP3 immune sera, could correspond to a degradation fragment of PP21.

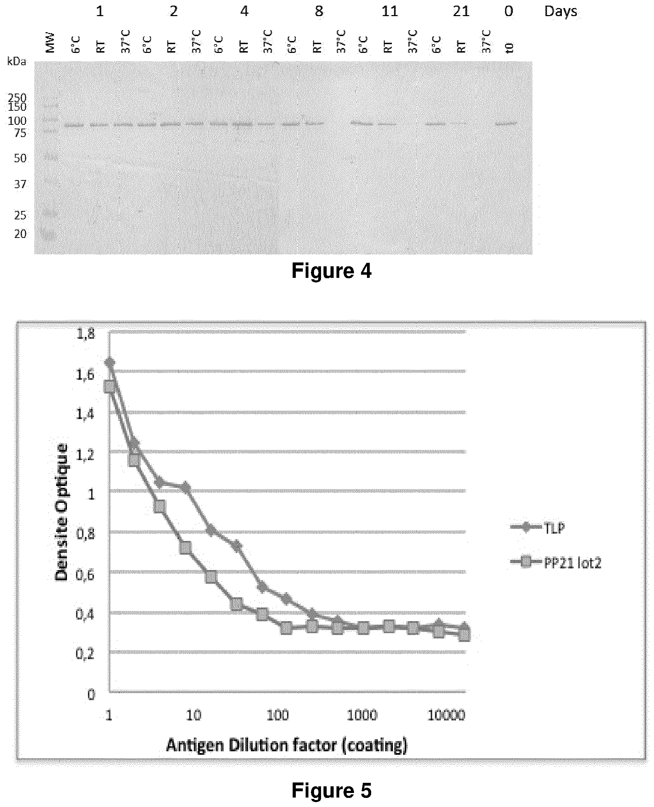

**Figure 4****:** Timescale of PP21 stability at different storage temperatures assessed by Western blotting with the anti-MSP3-1 mAb RAM1 at a 1:1000 dilution.
**Figure 5****:** Determination of the Antigen Content of PP21 as compared to the positive control MSP3-LSP. The antigenicity of pre-clinical PP21 was determined by reactivity with a large panel of human and animal anti-MSP3 sera, including a human recombinant antibody. The antigen content provides a more precise measurement of the quantity of reactive epitopes. It is performed by serial dilutions of the antigen, used for coating in an Elisa assay and revealed by a well-defined positive control (here a pool of rats immunized by the C-term region of MSP3). The reactivity of PP21 was identical to that of MSP3 synthetic and recombinant constructs

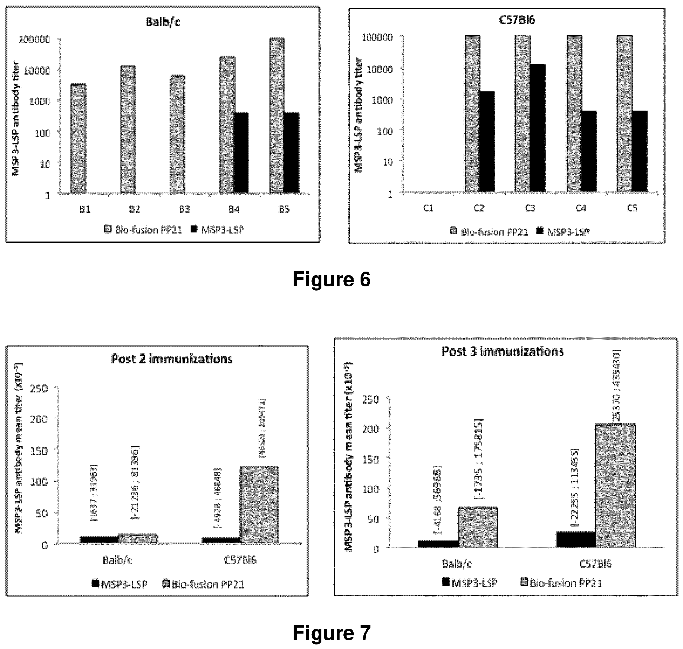

**Figure 6****:** Significant improvement in specific antibody response induced in mice following 2 immunizations by the Bio-fusion MSP3-1 conjugate as compared to the current MSP3-1-LSP. Groups of 5 (BALB/c) or 4 (C57BL/6) mice were immunized using either only 1 µg of bio-fusion MSP3-1 or 10 µg of MSP3-1 LSP in Montanide ISA720 on days 0, 14 and 28. Blood samples were collected one week after the 2^{nd} injection, on day 21 (A) and one week after the 3^{rd} immunization, on day 35. Specific MSP3-1 LSP antibody titers were determined by ELISA. The results for each mouse (1 to 5) are shown separately (here after only two immunizations).
**Figure 7****:** Comparison of antibody response induced in mice following 2 versus 3 immunizations by the Bio-fusion MSP3-1 as compared to the current MSP3-1-LSP. The third injection boosted the antibody response and confirmed the better immunogenicity of Bio-fusion MSP3-1 in C57Bl/6 mice and Balb/c mice . Mean titers of MSP3-1-LSP specific antibodies induced by 3 injections of 1 µg of Bio-fusion MSP3-1 reached 3×10⁵ and 2×10⁵ in C57Bl/6 and Balb/c respectively; whereas 3 injections of 10 µg of MSP3-1-LSP induced a maximum of 10⁵ and 2.5×10⁴ mean titers in C57Bl/6 and Balb/c mice respectively.

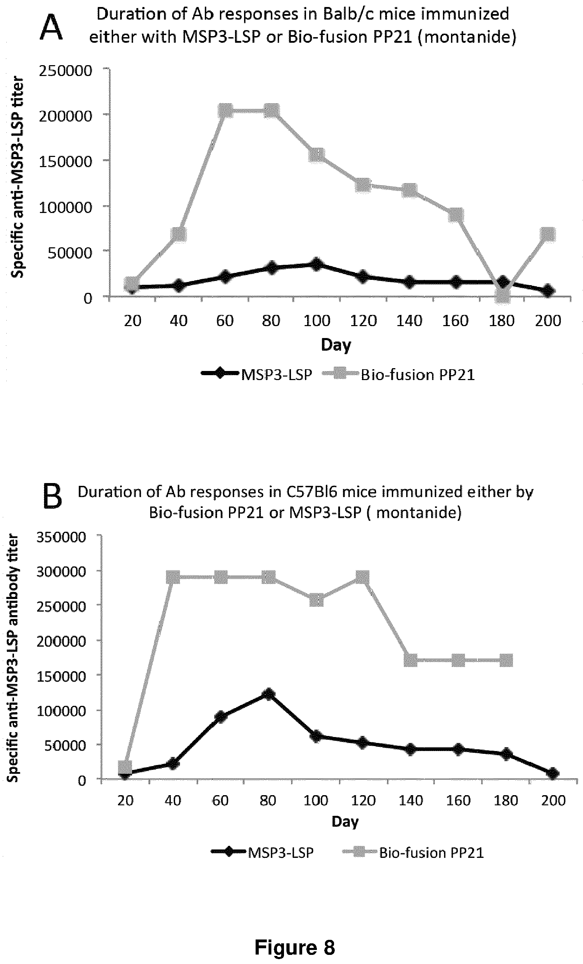

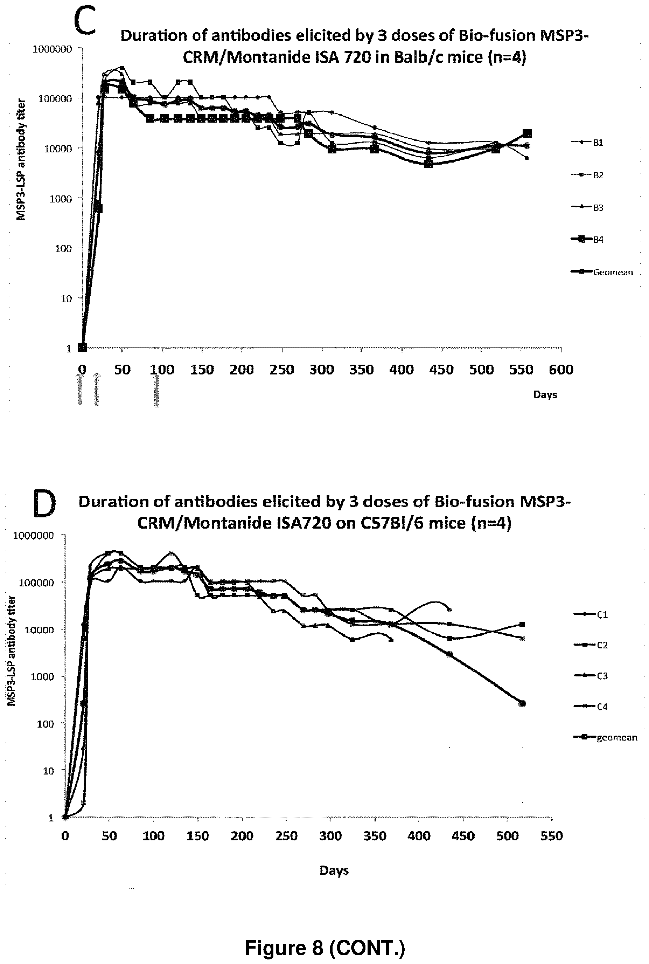

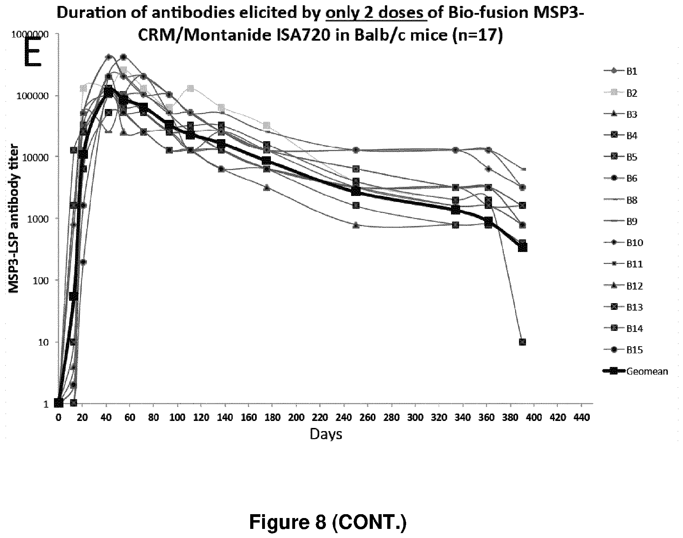

**Figure 8****:** Major improvement in duration of MSP3-1 specific antibodies induced in mice by the Bio-fusion MSP3-1 as compared to the current MSP3-1-LSP.
   A and B : comparison of Bio-Fusion MSP3-CRM with MSP3-1-LSP: BALB/c (A-upper left panel) and C57BL/6 (B-upper right panel) mice were immunized using either only 1 µg of Bio-fusion MSP3-1 or 10 µg of MSP3-1 LSP in Montanide ISA720 on days 0, 14 and 28. Blood samples were collected at different days over the follow-up. Specific MSP3-1 LSP antibody titers were determined by ELISA. The results shown correspond to the geometric mean of anti-MSP3-1 LSP antibody titers obtained in each group of mice at different days. After 160 days of follow-up the mean antibody titer in the Bio-fusion-MSP3-1 conjugate immunized mice was 1.5×10⁵ and 10⁵ in C57bl/6 and Balb/c respectively, which is at least 10 fold higher than the mean titer of antibodies induced by MSP3-1-LSP and measured at day 160 after the first immunization.
   C and D : Long-term persistence of immune responses elicited by MSP3-CRM: Groups of Balb/c and C57Bl6 mice (4 to 5 mice per group) were immunized by subcutaneous injection on days 0, 14 and 103 of 1µg of Bio-fusion MSP3-CRM adjuvanted in Montanide ISA 720 adjuvant (v/v). Blood samples were collected at different days. Specific MSP3 antibody titers were determined by ELISA. Results show that the titer increases remarkably after two immunizations and reaches at day 49 a maximum, whereas the third antigen injection, performed on day 103, did not induce any detectable increase in the magnitude of the antibody response. These specific antibodies decreased only slightly over time, and remained detectable at high titer even 540 days after immunization
   E: Long-term persistence of immune responses elicited by MSP3-CRM after only 2 immunisations. In view of satisfactory responses observed after only two immunisations, this immunization scheme was analyzed in further seventeen Balb/c mice immunized by subcutaneous injection on days 0 and 14 only, using 2µg of Bio-fusion MSP3-CRM adjuvanted in Montanide ISA 720 (v/v). Blood samples were collected at different days. Specific MSP3-1-LSP antibody titers were determined by ELISA. Results show that the MSP3 antibody titer increased after two antigen injections and reaches a maximum between days 42 and 72 in all mice, and show that immune responses decreased only modestly over the 380 days follow-up of the experiment.

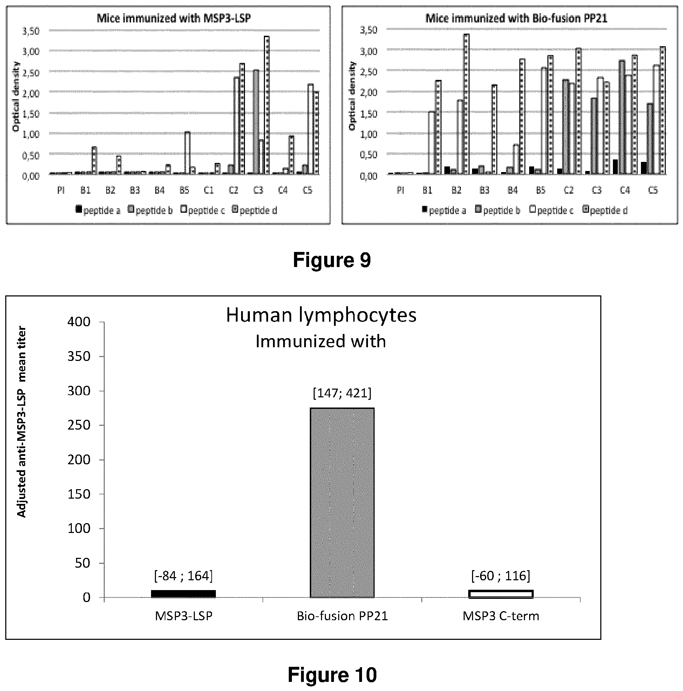

**Figure 9****:** Improved recognition of MSP3-1 ADCI (Ab-dependent cellular inhibition) target epitopes by antibodies induced in mice immunized with Bio-fusion MSP3-1 as compared to MSP3-1-LSP. In the MSP3-1-LSP, The peptides "b", "c" and "d" define distinct B-cell epitopes targeted by antibodies effective in the ADCI assay correlated to the protective effect of ant-MSP3-1 antibodies. To evaluate the ability of Bio-fusion MSP3-1 to induce antibodies to these epitopes we determined by ELISA the titers of antibodies to the peptides a, b, c and d in the sera. Hence the number of ADCI targets recognized by the novel formulation is considerably wider, and with higher titers. BALB/c and C57BL/6 mice were immunized using either only 1 µg of Bio-fusion MSP3-1 or 10 µg of MSP3-1 LSP in Montanide ISA720 on days 0, 14 and 28. Blood samples were collected one week after the 3^{rd} immunization, on day 35. The mice sera were diluted at 1/400 and the titer of specific antibodies of different MSP3-1 peptides (a, b, c and d) was determined by ELISA. The results shown correspond to the optical density obtained with the sera of each mouse tested.
**Figure 10****:** Human antibody responses in the HIMM model (Human Immunogenicicty Mouse Model) immunized either with 1 µg of Bio-fusion MSP3-1, 10 µg of the current MSP3-1 LSP or 10 µg of the MSP3-1 C-term recombinant protein. NOD.Cg - Prkdc^{scid} - IL2rg^{tm1WjI} /SzJ (NSG) mice were engrafted with human spleen cells stimulated or not in vitro with 1 µg/ml of antigen (4 mice per group). Each NSG mouse received an intraperitoneal injection (ip) of 30 × 10⁶ antigen-primed or unprimed spleen cells. At days 7 and 21, reconstituted mice were boosted with 1 µg of Bio-fusion MSP3-1, or 10 µg of MSP3-1-LSP or 10 µg of MSP3-1 C-term recombinant protein. All antigens were adjuvanted with Montanide ISA 720 (v/v) and injected intraperitoneally (ip). Mice reconstituted with unprimed spleen cells, received adjuvant alone. Blood samples were collected one week after each immunization. Specific antibodies were analyzed by total and specific IgG ELISA. The adjusted specific antibody titer was calculated using the following formula: (antigen specific antibody titer/total human IgG concentration) x 10 (10 mg/ml is considered as the mean human total IgG concentration). Geometric mean values (95 % CI) of ELISA titers are represented.

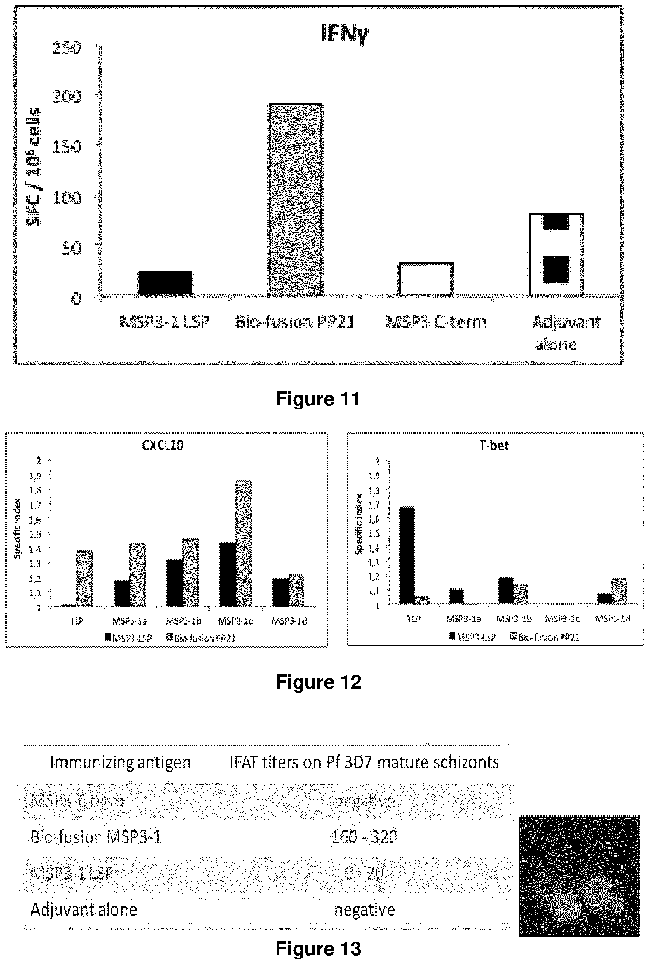

**Figure 11****:** Human IFN-γ cellular responses in the HIMM model (Hu Lymph NSG mice) immunized either with 1 µg of Bio-fusion MSP3-1, 10 µg of the current MSP3-1 LSP or 10 µg of the MSP3-1 C-term recombinant protein. Same immunizing conditions as above. Cellular responses were evaluated by IFN-γ ELISPOT. At day 28, spleen cells of each group of mice were recovered, pooled and cultured in vitro with or without antigen stimulation. The results shown correspond to the specific Spot Forming Cells (SFC) obtained with each group of mice.
**Figure 12****:** T-cell responses in HIMM: Marked improvement in CD4-Th1 responses in human lymphocytes grafted in mice immunized by either 1 µg of Bio-conjugate PP21 or 10 µg of MSP3-LSP.
**Figure 13****:** Improved recognition of parasite native proteins by human antibodies elicited in the HIMM model immunized with Bio-fusion MSP3-1. NSG mice were engrafted with human spleen cells and immunized either with 1 µg of Bio-fusion MSP3-1, 10 µg of the current MSP3-1 LSP or 10 µg of the MSP3-1 C-term recombinant protein (as described in the figure 6). The reactivity of human antibodies in day 28 mice sera, with the parasite proteins, which is a critical feature to activate the ADCI defense mechanism during a malaria attack, was evaluated by Immunofluorescence Antibody Test (IFAT) on 3D7 *Plasmodium falciparum* (Pf) infected red blood cells. The results shown correspond to the range of titers obtained with each group of mice.

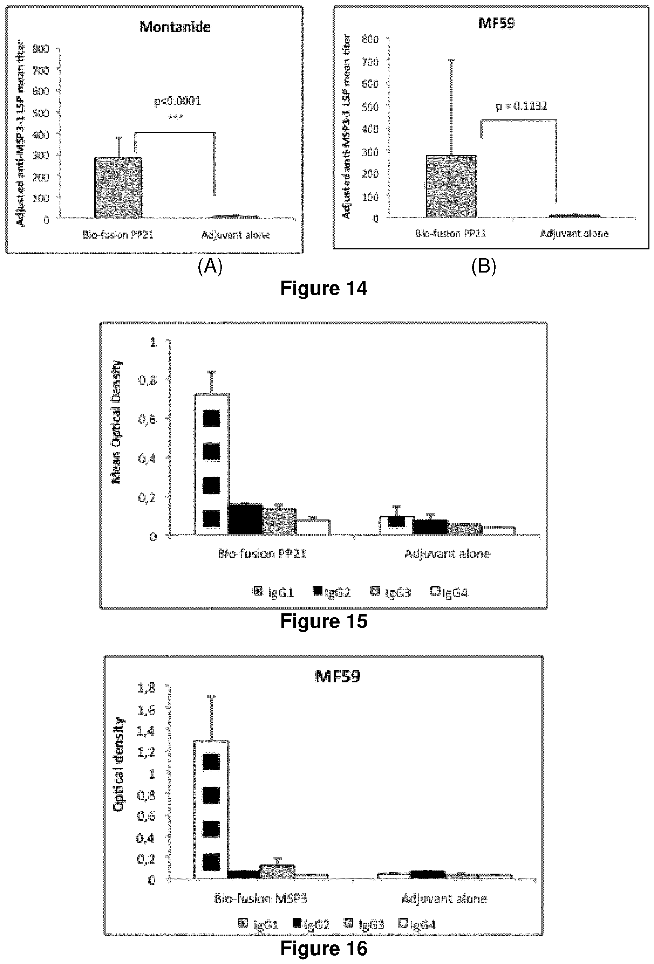

**Figure 14****:** The human antibody response elicited in the HIMM model immunized with 1 µg of Bio-fusion MSP3-1 does not depend on adjuvant used in the vaccine preparation. (A) NSG mice were engrafted with human spleen cells derived from two different donors and stimulated or not with 1 µg of Bio-fusion MSP3-1 adjuvanted with Montanide ISA720 (as described in the figure 6) (4 mice per group / donor). (B) NSG mice were engrafted with human spleen cells stimulated (9 mice) or not (8 mice) with 1 µg of Bio-fusion MSP3-1 adjuvanted with MF59 instead of Montanide ISA720 (as described in the figure 6 but with human lymphocytes from a distinct donor). In both cases, the specific MSP3-1 LSP antibody titers were determined by ELISA at day 28. The results shown correspond to the mean ± SD of the ELISA titers obtained with each group of mice.
**Figure 15****:** Antibody Subclasses of human IgG antibodies elicited in human lymphocytes in the HIMM model immunized with Bio-fusion MSP3-1 adjuvanted with Montanide ISA720. NSG mice were engrafted with human spleen cells stimulated or not with 1 µg of MSP3 Bio-fusion (as described in the figure 10). The mice sera, collected at day 28, were diluted at 1/20. The specific MSP3-1 LSP antibody titers were determined by subclass IgG ELISA. The results shown correspond to the mean ± SD of optical density obtained with immunized and non immunized mice.
**Figure 16****:** Subclasses of human IgG antibodies elicited in the HIMM model immunized with Bio-fusion MSP3-1 adjuvanted with MF59. NSG mice were engrafted with human spleen cells stimulated or not with 1 µg of Bio-fusion MSP3-1 adjuvanted with MF59 (as described in figure 14). The mice sera, collected at day 28, were diluted at 1/20. The specific MSP3-1 LSP antibody titers were determined by subclass IgG ELISA in the sera of responding mice. The results shown correspond to the mean ± SD of optical density obtained with immunized and non immunized mice. Both adjuvants led to obtain a dominance of the cytophilic subclass IgG1.

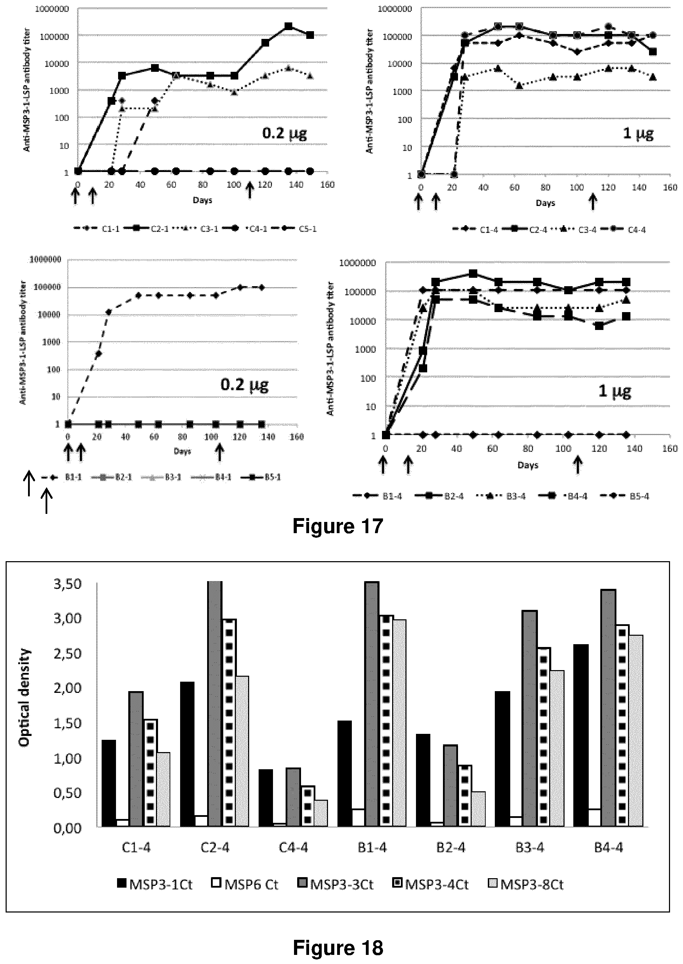

**Figure 17** **:** Immunogenicity of 0.2 or 1 µg of PP21 in Balbc mice /PP21-montanide (upper left and right), or the same doses in C57bl mice (lower left and right panels) Evolution of anti-MSP3-1-LSP antibody titers determined as in Figures 6-8
**Figure 18** : Cross-reactivity of anti-MSP3-1 Antibodies elicited by PP21 with other members of the MSP3 family of proteins (OD values by Elisa). C57Bl/6 (C) and Balb/c (B) mice received 2 injections (J0, J14) of 4µg PP21. Samples were collected on day 63.

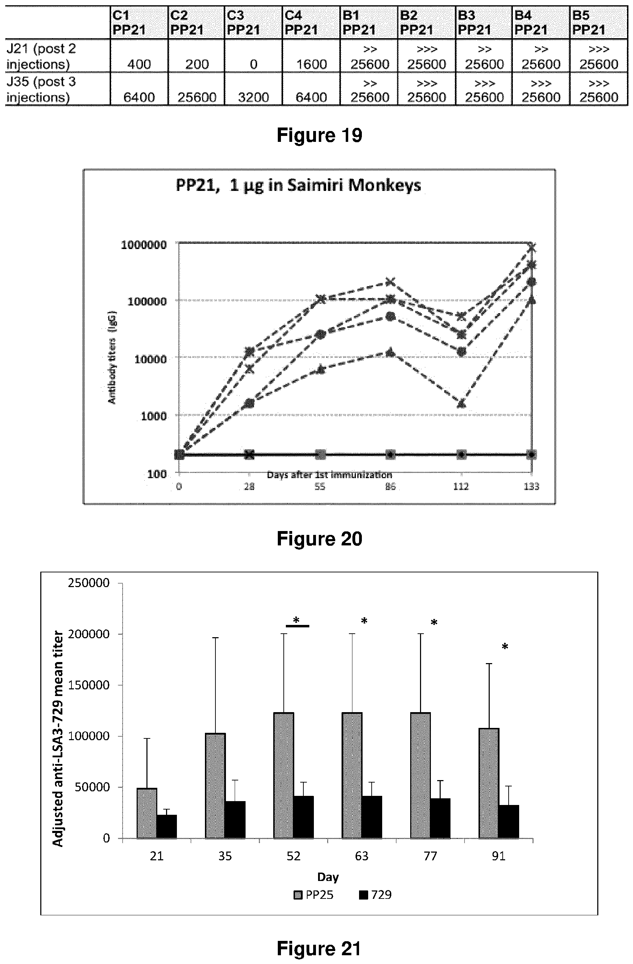

**Figure 19****:** Immunogenicity of PP21 adjuvated by Alum Hydroxyde in C57BL(C) and Balb/C mice(B), at a 1 µg dose per immunisation. Antibody Titers determined by Elisa in C57BL (C) and Balb/C mice (B), are shown following 2 immunisations or 3 immunisations. Higher dilutions than 25:600 were not tested. Note that Alum OH which is a potent adjuvant in humans yielding high Th1 responses, is in contrast usually a poor adjuvant in rodents, still induced potent responses with PP21.
**Figure 20****.** High Immunogenicity of the Bio-fusion conjugate PP21 in South-American Saimiri sciureus monkeys. Saimiri sciureus South American monkeys born in captivity in the animal house of the Primate Center of Belem, Brasil, were immunized by 1µg of PP21 adjuvated by Montanide Isa 720, administered SC on days O, 28 and 112 (dotted lines as compared to controls receiving Montanide alone, plain lines). Antibodies were determined by Elisa on the MSP3-LSP antigen, as well as by IFAT and WB (not shown). Even when using only 0.1µg antigen per immunisation, immune responses were about 5 fold lower, ie still very significant (not shown).
**Figure 21** : Antibody responses elicited by LSA3-CRM (PP25) versus LSA3-729 in Balb/C mice, receiving either 1µg of PP25 with Montanide ISA720 or 10µg of LSA3-729 in Montanide ISA720. Immunisations on day 0 ;7 ;28, serum collection on day 45.

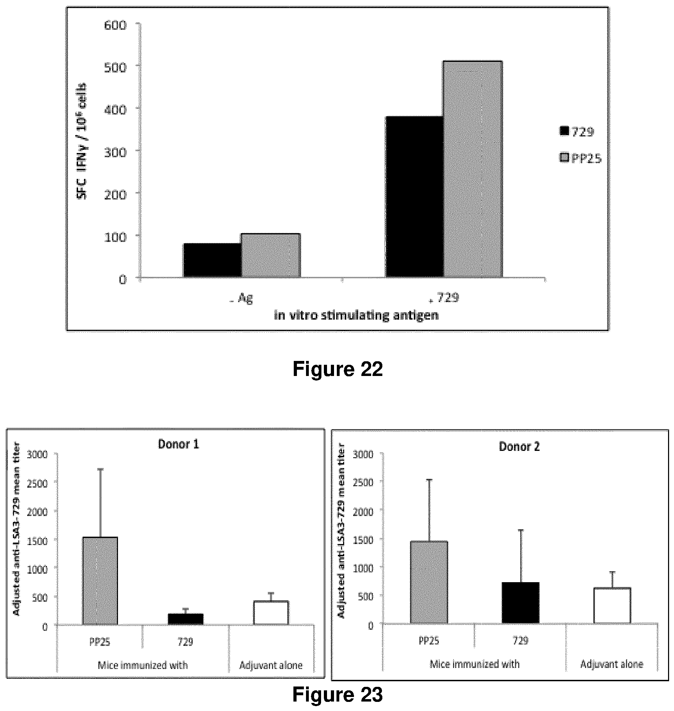

**Figure 22** **:** IFNγ (ELISPOT) elicited in LSA3-CRM (PP25) versus LSA3-729 immunized Balb/C mice, challenged in vitro by DG 729.
**Figure 23****:** Anti-LSA3-729 human antibody responses in HIMM and Reproducibility of the improvement of results: Anti-LSA3-729 human antibody responses in HIMM to the LSA3-CRM construct PP25 using lymphocytes from two human donors « A » and « B »

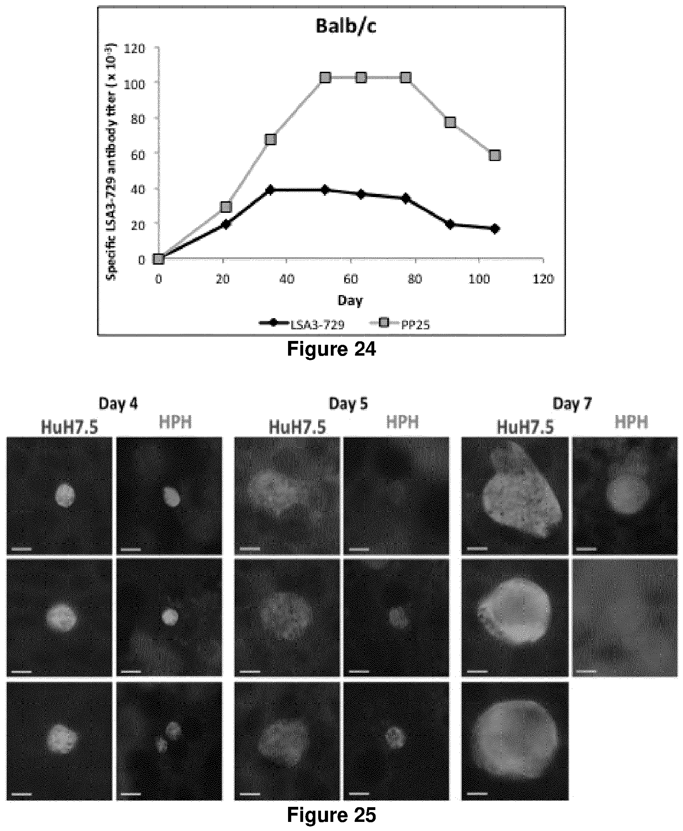

**Figure 24** **:** Improvement in the duration of immune responsees eelicited by PP25.
**Figure 25** **:** A novel host cell for P. falciparum liver stage development in HuH7.5 was developed and compared to Human Primary Hepatocytes (HPH). The figure shows representative samples of liver schizonts at 4, 5 and 7 days after infection with sporozoites, immunostained with a mouse polyclonal anti PfHsp-70. Magnification 400X, scale bar 10 µm. It was difficult to find satisfactory LS at day 7 in HPH. This hepatoma model was thereafter employed in a functional assay to measure antibodies able to inhibit the invasion of sporozoites into hepatocytes

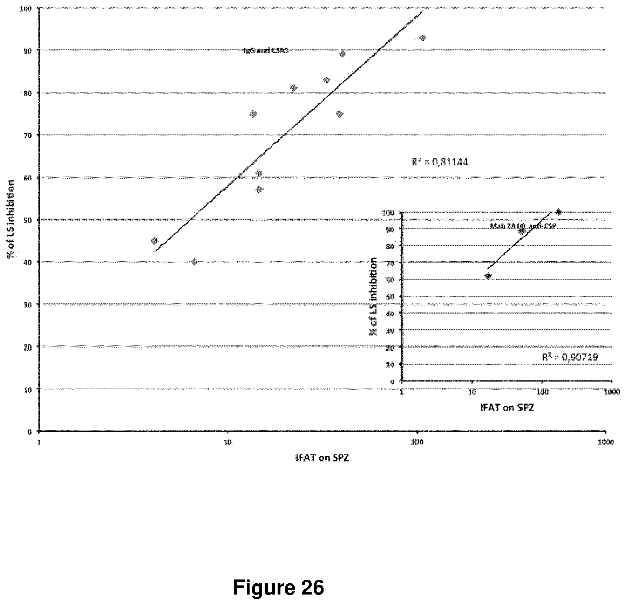

**Figure 26****:** Inhibition of sporozoite invasion in Hu7.5 hepatoma cells (ILSDA) by anti-Pf LSA3 antibodies. Shown is the correlation between anti-LSA3 reactivity on native proteins (by IFAT assays), with the functional inhibitory effect in the Inhibition of Liver Stages Development Assay (ILSDA). The smaller graph shows the same assays using the anti-Circumsporozoite mAb 2A10.

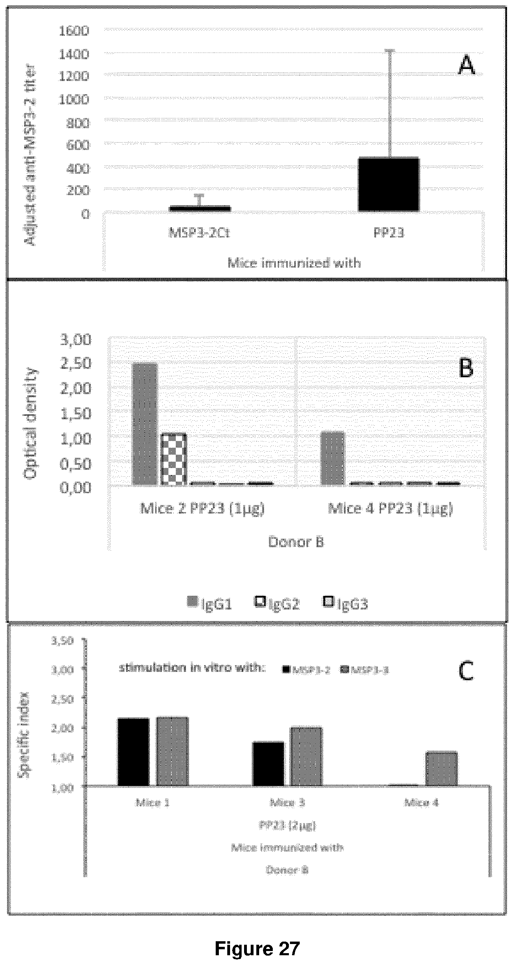

**Figure 27****:** Strong B and T cell responses were elicited in Human Lymphocytes grafted in the HIMM model, by the construct PP23 (MSP3-2-CRMP97-MSP3-3). The upper panel (A) shows the antibody responses, the middle panel (B) the isotypes distribution, which is predominantly of the IgG1 class , the main cytophilic class able to act in copperation with Monocytes in the ADCI mechanism. The lower panel (C) shows T-cells responses evaluated by secretion of IFN-γ restimulated in vitro by either MSP3-2 or MSP3-3.

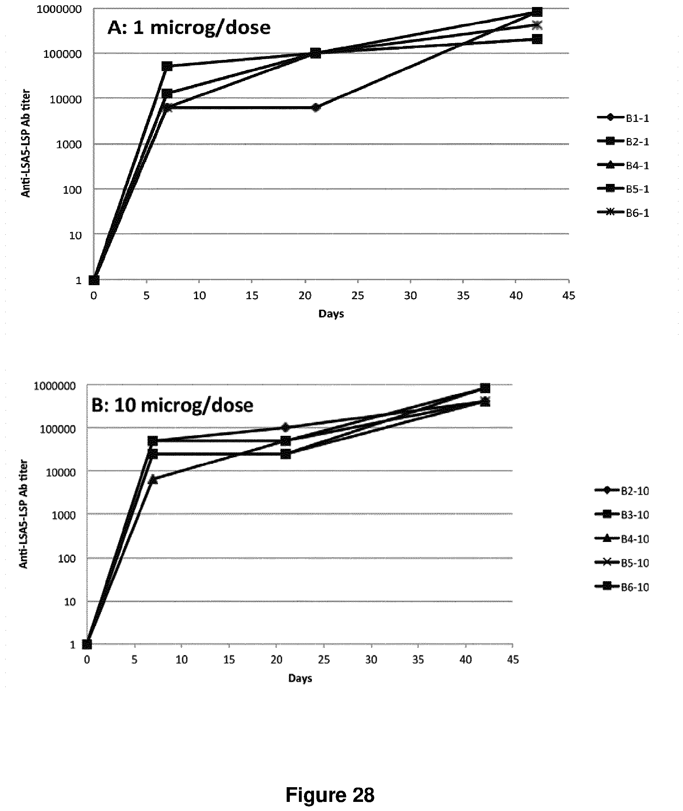

**Figure 28****:** Immunogenicity of 1 µg compared to 10 µg dose of Bio-fusion LSA5-CRM/Montanide ISA720 in BALb/c mice (n=5). Experimental conditions are similar to those described in Figure 8. Immunization by Bio-fusion LSA5-CRM /Montanide ISA720 was performed on days 0, 14 and 28 (arrows). Serum antibody detection was performed by ELISA titration on LSA5-LSP as antigen, using sera collected on days 0, 7, 21, 42.

### Detailed description of the invention

### Definitions

A "vaccine" is to be understood as meaning a composition for generating immunity for the prophylaxis and/or treatment of diseases. Accordingly, vaccines are medicaments which comprise antigens and are intended to be used in humans or animals for generating specific defense and protective substance by vaccination. A "vaccine," as used herein, means an immunogenic composition capable of eliciting partial or complete protection against malaria. A vaccine can be prophylactic for infection and/or therapeutic in an infected individual.

The term "immunogenic" means that the composition or protein to which it refers is capable of inducing an immune response upon administration. "Immune response" in a subject refers to the development of an adaptative and/or innate immune response, including a humoral immune response, a cellular immune response, or a humoral and a cellular immune response to an antigen. A "humoral immune response" refers to one that is mediated by antibodies. The term "protection" or "prevention" as used in this document refers to an absence of symptoms or to the presence of reduced symptoms of the disease after contact with the Plasmodium parasite. In particular, the term "prevention of malaria" refers to an absence of symptoms or to the presence of reduced symptoms in the treated subject after contact with a Plasmodium responsible for malaria.

The "patient" or "subject" is typically a mammal subject, preferably a human subject. The subject can be male or female, a child or an adult. A subject can be one who has been previously diagnosed or identified as having malaria. Alternatively, a subject can also be one who has not been previously diagnosed as having malaria, but who is at risk of developing such condition, e.g. due to infection or due to travel within a region in which malaria is prevalent. For example, a subject can be one who exhibits one or more symptoms for malaria. For instance the subject may be infected with Plasmodium falciparum or Plasmodium vivax. A "homologous" or "substantially homologous" sequence to an original polypeptide is typically one which has at least about 80 percent identity to the sequence of the original polypeptide or an immunogenic or epitopic fragment of the original polypeptide, and which substantially retains the desired effect on the intended target of the original polypeptide (i.e., elicits an immunogenic response, or being recognized by an antibody). In increasingly preferred embodiments, the homologous sequence has at least about 85 percent, 90 percent, 95 percent, 97 percent or 99 percent identity to the sequence of the original polypeptide or an immunogenic or epitopic fragment thereof.

An "immunogenic fragment" generally has a length of at least five amino acids. With increasing preference, the length of the immunogenic fragment is at least 7, 9, 11, 13, 15, 17 and 19 amino acids. An "epitope" generally has a length of at least five amino acids, preferably at least 7 to 14 amino acids.. An "epitopic fragment" comprises such epitope and can thus be longer.

In the context of the present invention, the term "fusion or biofusion" means that, in the recombinant protein, the carrier protein is either directly linked to the antigenic Plasmodium sequence, or is linked to the antigenic Plasmodium sequence by a peptide linker. The peptide linker may be a sequence of 1 to 35 amino acids, preferably 5 to 20 amino acids, still preferably 5 to 10 amino acids. In a preferred embodiment, the linker peptide is (Gly-Gly-Gly-Gly-Ser)n, (Gly)n or (EAAAK)n, wherein n is 1 to 4, preferably 1 to 3. In either embodiment, a single polypeptide is formed.

### The carrier protein

The carrier heterologous protein sequence may be advantageously selected from the group consisting of a cross-reacting material (CRM) of diphtheria toxoid, diphtheria toxoid (D), a non-toxic mutant recombinant of Pseudomonas aeruginosa exoprotein A (repA), meningococcal outer membrane protein complex (OMPC), tetanus toxoid (T), H. influenza protein D (hiD), and immunogenic fragments thereof.

In preferred embodiments, the carrier heterologous protein sequence may be CRM197 or an immunogenic fragment thereof, which fragment is preferably selected from the group consisting of Fragment A, Transmembrane Domain T, Receptor Binding domain R of CRM197, amino acid sequence 1-190 of CRM197, and amino acid sequence 1-389 of CRM197.

Fragment A of diphterin toxin extends from amino acid 1 to 201. CRM197 possesses an enzymatically inactive fragment A, showing a substitution of glycine 52 with glutamic acid. Fragment B of diphterin toxin extends from amino acid 202 to 535.

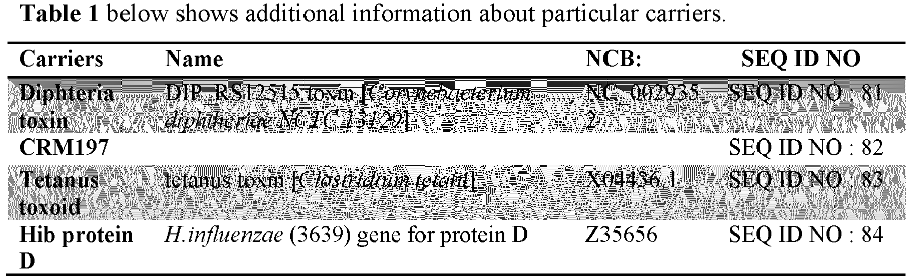

Substantially homologous sequences may also be used.

### The antigenic Plasmodium sequence

The fusion protein construct of the invention comprises at least one antigenic sequence comprising an epitope sequence of a Plasmodium protein.

Said antigenic sequence may be of any Plasmodium antigen, from any Plasmodium species.

The "Plasmodium" genus of parasites infecting humans include, without limitation, P. falciparum, P. vivax, P. knowles, P. ovale and P. malariae. The antigens listed below are mostly antigens from P. falciparum. However orthologs in other Plasmodium species are encompassed as well. In particular P. vivax antigens have shown the same low immunogenicity as P. falciparum antigens.

Said antigen may be expressed in erythrocyte stage (also designated "blood stage") or pre-erythrocytic stage (sporozoites and liver stages).

Examples of antigenic sequences which may be used are listed in **Table 2** below. Substantially homologous sequences may also be used.

Antigens expressed in erythrocyte stage, which are target of Ab-dependent cellular inhibition (ADCI) defense mechanism include:
MSP3 (e.g. MSP3-1, MSP3-2, MSP3-3, MSP3-4, MSP3-7, MSP3-8), PEBS sub-region of 11-1 (also named LSA5), Glurp R0 and R2, SERP, P27 and P27A, P45, P90 and P77, P14, P181 (which is a combination of P77), MSP1-Block 2, MSP2 (two 3D7 and FC27 alleles, both dimorphic and constant regions), GBP 130 (especially both N and C moieties), protein 332, protein 11-1, MSP4, or any epitopic fragment thereof.

Preferably the antigenic sequence in the fusion protein is MSP3 or an epitopic fragment thereof.

In another preferred embodiment, the antigenic sequence in the fusion protein is LSA5 or an epitopic fragment thereof.

Plasmodium falciparum merozoite surface protein 3 (MSP-3) is a known asexual blood-stage malaria vaccine candidate antigen (Sirima et al, N Engl J Med 2011, 365(11):1062-1064; Demanga et al, Infect Immun 2010, 78(1):486-494; Daher et al, Infect Immun 2010, 78(1):477-485; Rousillon et al, Roussilhon, et al, PLoS Medicine, November 2007, 4, Issue 11, e320).

In particular any of the six MSP3 proteins may be used, preferably MSP3-1, as well as peptides a,b,c,d, e,f,, the recombinants C-term, but also full length and N-term of the protein. In a preferred embodiment, the antigenic sequence is MSP3 or an epitopic fragment thereof, which fragment encompasses motifs a, b, c, d, e and/or f of the C-terminal region of a Plasmodium MSP3 protein chosen among MSP3-1, MSP3-2, MSP3-3, MSP3-4, MSP3-7, MSP3-8.

Other antigens from the merozoite surface, particularly those loosely attached or cleaved and therefore which can be released from the merozoite surface, are encompassed.

Antigens expressed in the pre-erythrocytic stage include LSA3, PEBS, CS, Trap, and Salsa, and any epitopic fragment thereof. In P. falciparum, liver stage antigen-3 (LSA-3) is an antigen expressed at the pre-erythrocytic stage (Toure-Balde et al: Infect Immun 2009, 77(3):1189-1196; Daubersies et al: Nat Med 2000, 6(11):1258-1263).

Antigens involved in other stages or other mechanisms are also encompassed. Those include antigens involved in the sexual stages, e.g. Pf25 or Pf45/48, and antigens of blood stage involved in GIA mechanism of inhibition of merozoite invasion in red blood cells, such as Rh5, AMA1, MSP1-19, MSP1-42, MSP2 or MSP4-5, or involved in cyto-adherence, such as Var CSA.

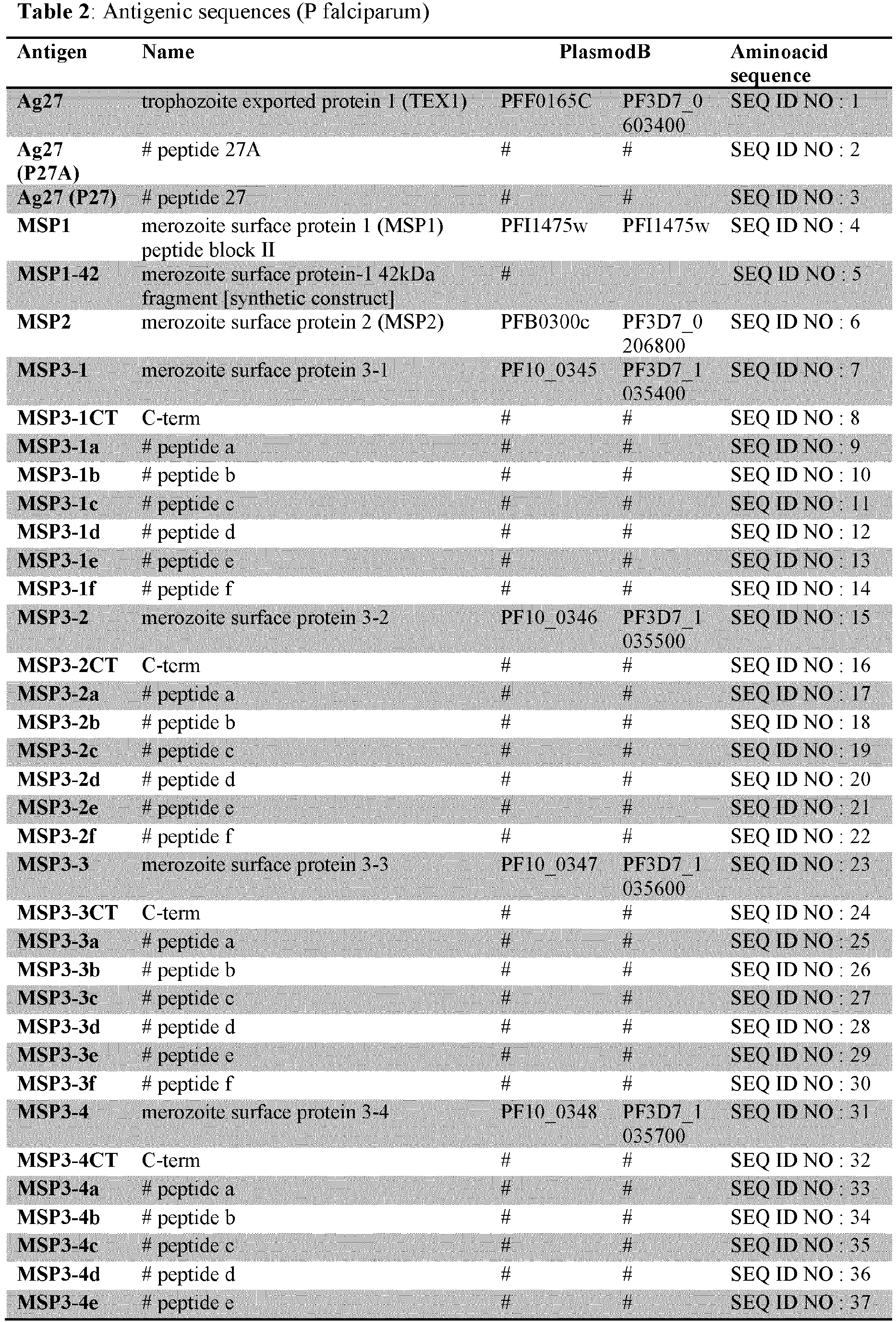

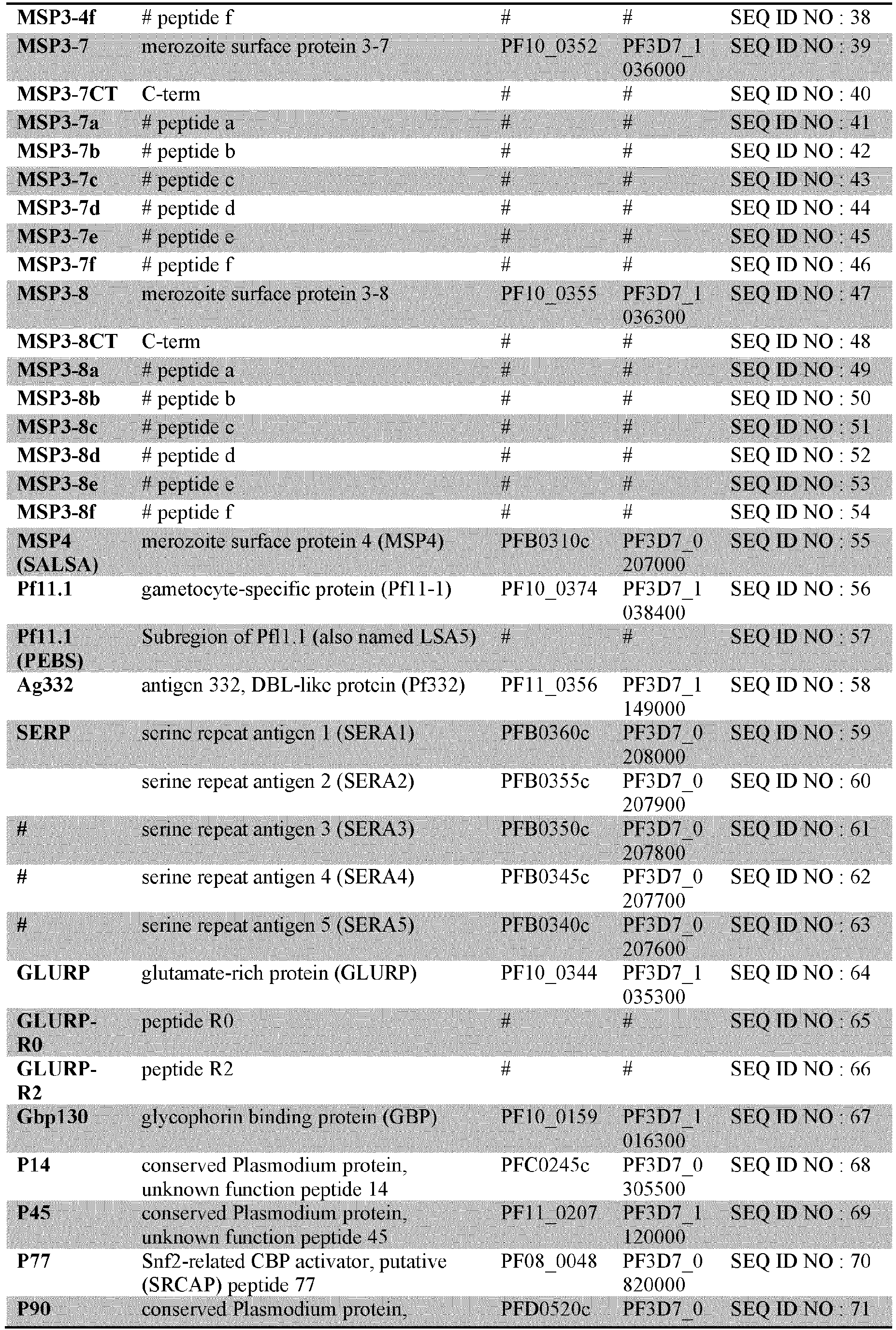

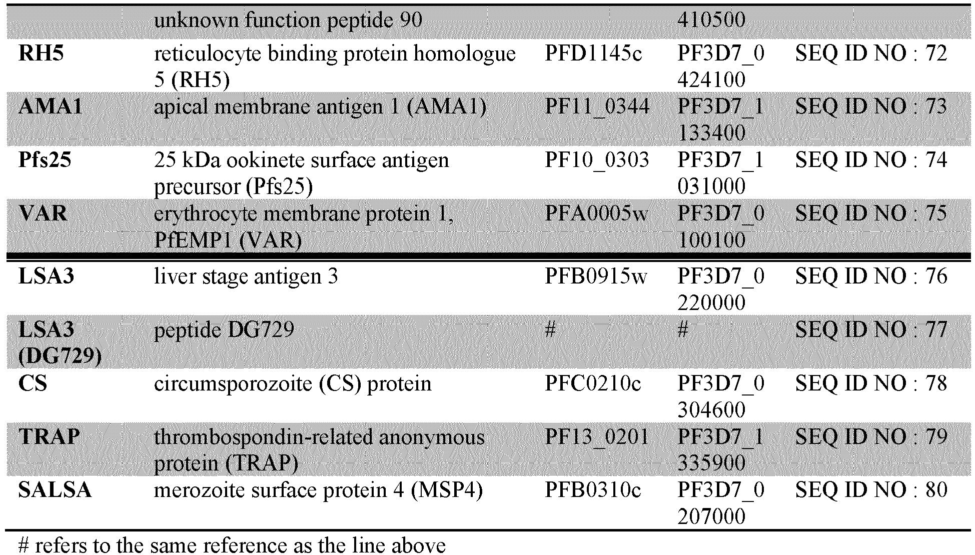

### The fusion construct

In a particular embodiment, the fusion protein comprises or consists of MSP3 (preferably MSP3-1), LSA3, LSA5 (also named PEBS) or an epitopic fragment thereof, as the antigenic sequence, fused to CRM197 or an immunogenic fragment thereof, as the carrier sequence.

In a preferred aspect, the fusion protein comprises two antigenic sequences which are either each located in C-term and N-term of the carrier protein sequence, or both located at the same terminus, wherein the two antigenic sequences are the same or different from each other.

In a preferred embodiment, the fusion between the antigenic sequence and the carrier sequence is a direct fusion.

In another embodiment, the at least one amino acid sequence may be linked at the N-term and/or C-term of the carrier heterologous protein sequence by a peptide linker, wherein the peptide linker preferably is a sequence of 1 to 35 amino acids, preferably 5 to 20 amino acids. In a preferred aspect, the peptide linker is (Gly-Gly-Gly-Gly-Ser)n, (Gly)n or (EAAAK)n, wherein n is 1 to 4, preferably 1 to 3.

### Acid nucleic, vectors and host cells

The fusion protein of the present invention can be made by any recombinant technique. Accordingly, another aspect of the invention pertains to vectors, preferably expression vectors, containing a nucleic acid encoding said fusion protein.

The expression vectors used in the present invention can provide for expression in vitro and/or in vivo (e.g. in a suitable host cell, host organism and/or expression system). They typically comprise a polynucleotide sequence as defined above, and regulatory sequences (such as a suitable promoter(s), enhancer(s), terminator(s), etc.) allowing the expression (e.g. transcription and translation) of the protein product in the host cell or host organism.

The vectors according to the invention may be in the form of a vector, such as for example a plasmid, cosmid, YAC, a viral vector or transposon.

In a preferred but non-limiting aspect, a vector of the invention comprises i) at least one nucleic acid as described above; operably connected to ii) one or more regulatory elements, such as a promoter and optionally a suitable terminator; and optionally also iii) one or more further elements of genetic constructs such as 3'- or 5'-UTR sequences, leader sequences, selection markers, expression markers/reporter genes, and/or elements that may facilitate or increase (the efficiency of) transformation or integration.

A preferred host cell is E.coli. However any prokaryotic or eukaryotic expression systems may be used, such as bacteria, yeast, filamentous fungi, insect, plant cells and mammalian cells.

Preferred vectors, promoters, and host cells, are all vectors, promoters and host cells able to express the recombinant protein, glycosylated or not-glycosylated, such as: the pET26a (with T7 promoter), or the pTrcHis2 (with trp lac promoter) plasmids for procaryotic expression, the pUC19 , pUC 57, or pCR4 TOPO plasmids for transfer, and the E. coli BL21 (DE3), or E coli NiCo21 host cells for protein expression, or E coli Top 10 only for plasmids amplification.

### Vaccine formulation

The fusion protein is formulated in a pharmaceutical composition, in association with a physiologically acceptable vehicle, optionally combined with an adjuvant.

It is therefore provided a vaccine comprising the fusion protein, or a combination of at least two fusion proteins, which respectively comprise at least two different antigenic amino acid sequences, with a physiologically acceptable vehicle.

In another embodiment, it is provided a vaccine composition comprising a nucleic acid construct encoding said fusion protein(s).

In some embodiments, the vaccines may comprise one or more pharmaceutically acceptable vehicles or excipients. Excipients include any component that does not itself induce the production of antibodies and is not harmful to the subject receiving the composition. Suitable excipients are typically large, slowly metabolized macromolecules such as saccharides, polylactic acids, polyglycolic acids, polymeric amino acids, amino acid copolymers, sucrose, trehalose, lactose and lipid aggregates (such as oil droplets or liposomes). Suitable pharmaceutical vehicles are well known to those of ordinary skill in the art, including, but not limited to, diluents, such as water, saline, and others. Suitably, sterile pyrogen-free, phosphate buffered physiologic saline is a pharmaceutical vehicle. Additionally, additives, such as wetting or emulsifying agents, pH buffering substances, and the like, may be present.

The pharmaceutical composition, immunogenic composition or vaccine may further comprise an adjuvant. "Adjuvants" are used to enhance efficacy of the composition and include, but are not limited to, aluminum hydroxide (alum), aluminum phosphate, oil-in-water or water-in-oil emulsions, agonists of Toll-like receptors, N-acetyl-normuramyl-L-alanyl- D-isoglutamine (the-MDP), N-acetyl-muramyl-L-threonyl-D-isoglutamine (nor-MDP), PEI; AS01 or AS02 (GlaxoSmithKline), GLA-SE (IDRI) and similar formulations, ; and the like adjuvants known in the art. The Montanide^{®} adjuvants which are based on purified squalene and squalane, emulsified with highly purified mannide mono-oleate, may be used as well, including ISA 51 and 720. MF59^{®} is another example of an adjuvant, which is an oil-in-water emulsion of a squalene, polyoxyethylene sorbitan monooleate (Tween^{®} 80) and sorbitan trioleate.

Vaccines are formulated into suitable dosage for the subject to which it is to be administered. The dosage administered may vary with the condition, sex, weight and age of the individual; the route of administration; and the adjuvant used. The vaccine may be used in dosage forms such as suspensions or liquid solutions. The vaccine may be formulated with a pharmaceutically acceptable vehicle as described above. Suitable dosages include, but are not limited to, about 0.1 to about 100 micrograms, preferably about 1 to about 50 micrograms, still preferably 1 to 20, 1 to 15, or even 1 to 10 micrograms, of the fusion protein described herein.

### Vaccination

The immunogenic composition or vaccine may be a multi-component/multi- antigen immunogenic composition or vaccine.

Where the immunogenic composition or vaccine is intended for a multiple administration regime, such as a prime-boost regime, the prime composition may comprise the same or different composition as the boost composition. However a prime-boost regime is not compulsory at all in the context of the present invention.

The immunogenic composition or vaccine may be administered by any convenient route, preferably parenterally, intramuscularly, intradermally, subcutaneously, mucosally, or intravenously.

A variety of techniques are available for DNA vaccination, such as electroporation, needle-free approaches, such as particle bombardment and high-pressure delivery, dermal patches, formulation of DNA vaccine in microparticles or liposomes.

It is described a method for raising an immune response in a subject, comprising the step of administering an effective amount of a vaccine of the invention. The vaccines can be administered prophylactically (i.e. to prevent infection) or to provide protective and preferably involves induction of antibodies and/or T cell immunity. The method may raise a primary immune response, a secondary immune response, a booster response or a combination of immune responses.

The vaccine is meant to protect the subject against malaria at any stage, including pre-erythrocytic-stages, such as liver-stage, and/or sexual or asexual blood-stage.

The magnitude and/or memory of the immune response against the Plasmodium antigens is increased according to the invention.

Especially, the MSP3 fusion constructs of the invention trigger production of anti-MSP3 cytophilic antibodies such as IgG1 and IgG3 antibodies. The level of protection may be determined by measuring the titer of said antibodies.

The Figures and Examples illustrate the invention without limiting its scope.

### EXAMPLES:

Previous studies have shown that frequent malaria exposure leads to the development of immunity in adults, which prevents or alleviates disease. This immunity can be passively transferred by administering immunoglobulin (IgG) from malaria-immune adults.

A parasite-killing mechanism, called antibody-dependent cell-mediated inhibition (ADCI), was identified. The screening of a genome-wide DNA library by ADCI led to the identification of merozoite-surface-protein 3 (MSP3) and later to several other antigens, including PEBS.

Cytophilic antibodies were identified as surrogates of protection: Cytophilic (leucocyte-binding) anti-MSP3 antibodies IgGI and IgG3, which mediate ADCI were found to be consistently associated with a reduction in the risk of malaria in epidemiological studies in many different settings. These antibodies also mediate protection against P.falciparum in infected animals.

These studies indicate the characteristics which an efficient immune response bears and guide the development of improved vaccine formulations.

On this basis, the inventors have now shown that a fusion protein CRM97-MSP3 increases immunogenicity by 20-100 fold, at B and T-cell levels, as compared to MSP3-LSP (long synthetic peptide covering the 186-271 region of MSP3-1). See **Example 1.** Using 10 fold lower immunizing doses, it translated into a major improvement in immunogenicity to lymphocytes from : humans, mice and saimiri monkeys, and a major improvement in duration of antibodies.

At pre-erythrocytic stage, previous studies have further shown that protective efficacy against massive sporozoites challenges can be elicited by LSA3 in Chimpanzees, which efficacy depends on IFN-γ likely inhibiting LS development, and on antibodies preventing sporozoite invasion.

In **Example 2,** the inventors have shown that a bio-fusion CRM-LSA3 construct strongly increases T and B cell responses.

The results obtained in Examples 1 and 2 proved to be reproducible using other antigens from the erytrocytic stages such as MSP3-2 and MSP3-3 in a construct with CRM (See **Example 3).**

**Example 4** provides results with a LSA5-CRM construct.

### Materials and methods

### Generation of expression constructs and production of the Bio-fusion proteins in Escherichia coli

The genetic sequences coding for the chimeric immunogens composed of the detoxified diphtheria toxin CRM197 covalently linked at both its N- and C-terminal end to malaria peptide antigens (Figure 1) were chemically synthetized (GenScript) and codon-optimized for the production in *E. coli.* Restriction sites for Nco I and Xho I were respectively added at the 5' and 3' extremities for cloning in the expression plasmid pET-26a (Novagen). The sequences inserted in the shuttle plasmid pUC57 were checked by sequencing and expedited lyophilized (4 µg).

The MSP3-1 peptide in PP21 (plasmodB acc. Nbr PF3D7_1035400) corresponds to amino acids (AA) 155 to 249 in MSP3-1.

Other hybrid immunogens (Table 3) have been designed around CRM197. In PP22, the MSP3-1 peptide was replaced by a MSP3-2 peptide (AA 178 to 257 of MSP3-2, PlasmodB acc. Nbr PF3D7_1035500). In PP23, the MSP3-2 peptide at the C-extremity was replaced by an MSP3-3 peptide (AA 246 to 307 in MSP3-3, PlasmodB acc. Nbr PF3D7_1035600). PP25 contains the same LSA3 peptide on both CRM197 extremities (AA 176 to 325, PlasmodB acc. Nbr PF3D7_0220000).

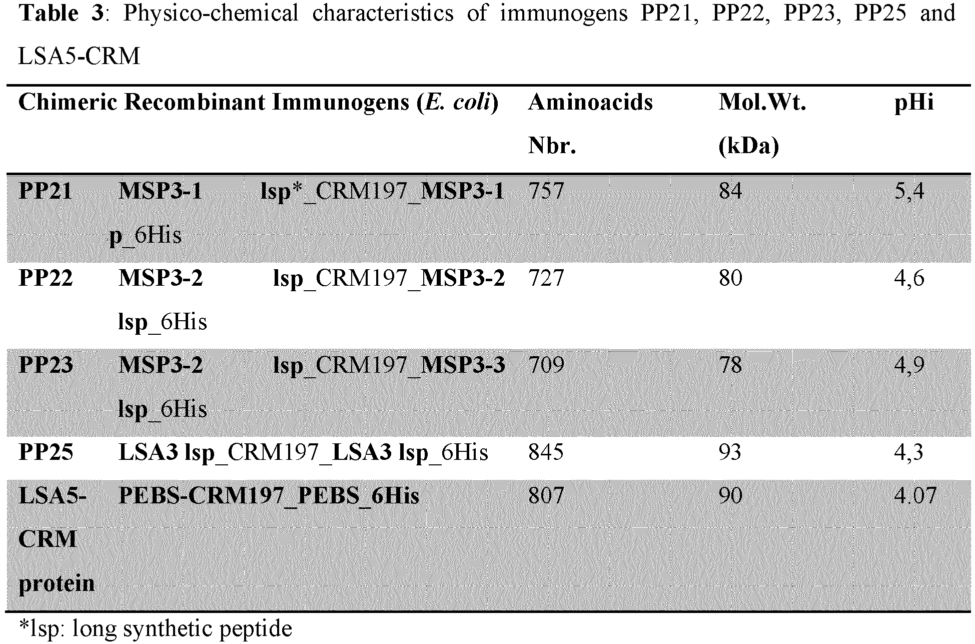

The *E. coli* transfer strain Top 10 (Invitrogen) [F- *mcr*A Δ(*mrr*-*hsd*RMS-*mcr*BC) Φ80*lac*ZΔM15 *ΔlacX74 rec*A1 *araD139* Δ(*ara leu*) 7697 *gal*U *gal*K *rps*L (StrR) *end*A1 *nup*G] was used as a for the cloning molecular procedures and plasmid propagation. Expression of recombinants was performed in strain BL21 (DE3) [F - *ompT hsdSB* (*rB*-*mB*) *gal dcm* (DE3)] (NEB).

The shuttle plasmid pUC57 containing the hybrid sequences (Genscript) and the expression plasmid pET26a were digested with Nco I and Xho I. The bands for the inserts and the linearized pET26a were recovered by gel extraction (QIAGEN) after electrophoresis. The gel-purified inserts were ligated inside pET26a with a ratio insert on plasmid of 3. The ligation reaction was used to transform chemically competent *E. coli* Top 10 and plated on LB agar media with 50µg/mL kanamycin in Petri dishes.

Six bacteria colonies were picked in liquid media with kanamycin for plasmid minipreparations on silica column (QIAGEN). The correct insertion of inserts in pET26a was checked by digestion with informative restriction enzymes **(****Figure 2****).**

Plasmids with the correct restriction profile were selected to transform BL21 (DE3) bacteria. Transformation with the correct plasmid was confirmed on six BL21 (DE3) clones that were analyzed by restriction enzyme digestion as above.

### Optimization of the recombinant protein production in small volume bacteria cultures

Parameters such as the duration of recombinant protein production, the concentration of the inducer (IPTG) and temperature were analyzed on a small culture scale (1 L) in Erlenmeyer vessels. Five mL of a steady state overnight culture was diluted in 1 L (1:200) and further cultured at 30 °C to an optical density of 0,7-0,8 at 600nm. The induction of the recombinant protein expression was assayed with two IPTG inducer concentrations; 0.1 and 1 mM at three temperatures; 22, 30 and 37°C. The best recombinant yields were obtained after on overnight culture at 22°C and induction by 0.1 mM IPTG.

A yield of 1 to 2 mg of recombinant product per liter of bacteria culture was obtained after purification by immobilized metal affinity chromatography (IMAC).

### Production and purification of PP21

Productions at a research laboratory scale (1 to 2 liters) were conducted to obtain sufficient PP21 recombinant protein to conduct pre-clinical immunogenicity studies in murine and primate models.

The protocol for the bacteria culture and recombinant protein production has been established formerly in small scale cultures.

The C-terminally hexa-histidine tagged recombinant protein PP21 was purified by affinity on Ni-NTA resin (QIAGEN).

Two purification procedures, under native or denaturing conditions, were investigated.

Indeed, although in native conditions, no recombinant protein was seen in the bacteria lysate insoluble fraction, purification under denaturing conditions was also assayed to assess the effect of a putative conformational effect of the tri-dimensional structure of PP21 on the availability of the histidine tag for the immobilized Ni²⁺ metal on the Ni-NTA resin.

### Purification under native conditions:

- The bacteria cells were recovered by centrifugation (4000g, 30 mins, 4°C).
- The bacteria pellet was suspended in 5 mL of lysis buffer (50 mM NaH₂PO₄, 300 mM NaCl, 10 mM imidazole, pH 8.0) per gram of biomass.
- The lysate was rocked on ice 30 mins with 1 mg/mL of lysozyme.
- The bacteria were lysed by 5 30 sec bursts at 150 W with a 30 sec cooling intervals.
- The lysate was rocked on ice 30 min with 5µg/mL of DNAse I and 10µg/mL of RNAse A with a complete set of proteases inhibitors (Roche).
- The lysate was cleared by centrifugation at 10000g, 30 mins at 4°C.
- The lysate was filtered through a 0,22 µm sieve and incubated overnight on a rocking tray at 4°C with 200 µL of Ni NTA resin in a 20 mL volume.
- The resin was extensively washed in a 40 mL volume of lysis buffer five times. An aliquot of the first step supernatant (lysate post-resin incubation) was conserved for analysis. Centrifugation steps did not exceed 5 mins at 200g.
- The recombinant proteins were recovered by incubating the resin in 500 µL lysis buffer containing 250 mM imidazole 5-fold
- The eluted fractions were pooled and dialyzed against PBS pH 7.3 by ultrafiltration through a 30 kDa sieve.

The protein concentration was measured by spectrophotometry at 280 nM and further characterized by electrophoresis (SDS-PAGE 10%) and immunoblotting as shown in Figure 3 for PP21.

### Purification under denaturing conditions:

The bacteria cells were recovered by centrifugation (4000g, 30 mins, 4°C).

The bacteria pellet was suspended in 5 mL of denaturing lysis buffer (100 mM NaH2PO4, 10 mM Tris-Cl, 8 M urea, NaOH pH 8.0) per gram of biomass.

The lysate was rocked on ice 30 mins.

The bacteria were further lysed by 5 30 sec bursts at 150 W with a 30 sec cooling intervals.

The lysate was cleared by centrifugation at 10000g, 30 min at 4°C.

The lysate was filtered through a 0,22 µm sieve and incubated overnight on a rocking tray at 4°C with 200 µL of Ni NTA resin in a 20 mL volume.

The resin was extensively washed in a 40 mL volume of denaturing lysis buffer five times. An aliquot of the first step supernatant (lysate post-resin incubation) was conserved for analysis. Centrifugation steps did not exceed 5 min at 200g.

The recombinant proteins were recovered by incubating the resin in 500 µL lysis buffer containing 250 mM imidazole 5-fold.

The eluted fractions were pooled and dialyzed against PBS pH 7.3 by ultrafiltration through a 30 kDa sieve.

The purification yield was similar by the two methods, suggesting that the affinity histidine tag is functional in the native form of PP21.

Production yields of 0,7 to 1mg of the recombinant protein PP21 per liter of bacteria culture have been reproducibly obtained in more than 10 production / purification trials at the 1 liter scale in Erlenmeyer vessels.

**Figure 3** shows recombinant protein PP21 purified by IMAC.

### Temperature stability of PP21

The stability of PP21 in function of the storage temperature assessed by *Western blotting* is shown in **Figure 4****.** No signs of degradations were observed after a period of 21 days storage in a fridge at a temperature of 6°C. At room temperature, PP21 started to show signs of degradation at the same period whereas it was completely degraded at 37°C after 8 days. However, this instability cannot be attributed to putative intrinsic molecule instability as it is not a GMP manufactured product and it might contain traces of proteases.

Amino acid sequences of recombinant proteins expressed in *E.coli* by pET26a are shown below. Sequences in brackets come from the expression plasmid, bold from the CRM197 diphtheria toxin.

The hexa-histidine tag at the C-terminal used for purification by immobilized metal affinity chromatography (IMAC) is underlined.
**PP21** sequence of MSP3-1 in italics-underlined (PlasmodB ID : PF3D7_1035400), residues 155 to 249 (95 aa) 
[MKYLLPTAAAGLLLLAAQPAMAMG]KTKEYAEKAKNAYEKAKNAYQKANQAVLKAKE
ASSYDYILGWEFGGGVPEHKKEENMLSHLYVSSKDKENISKENDDVLDEKEEEAEETEEEE
LEGADDVVDSSKSFVMENFSSYHGTKPGYVDSIQKGIQKPKSGTQGNYDDDWKE
 
FYSTDNKYDAAGYSVDNENPLSGKAGGVVKVTYPGLTKVLALKVDNAETIKKE
LGLSLTEPLMEQVGTEEFIKRFGDGASRVVLSLPFAEGSSSVEYINNWEQAKALS
VELEINFETRGKRGQDAMYEYMAQACAGNRVRRSVGSSLSCINLDWDVIRDKT
KTKIESLKEHGPIKNKMSESPNKTVSEEKAKQYLEEFHQTALEHPELSELKTVTG
TNPVFAGANYAAWAVNVAQVIDSETADNLEKTTAALSILPGIGSVMGIADGAVH
HNTEEIVAQSIALSSLMVAQAIPLVGELVDIGFAAYNFVESIINLFQVVHNSYNRP
AYSPGHKTQPFLHDGYAVSWNTVEDSIIRTGFQGESGHDIKITAENTPLPIAGVLL
PTIPGKLDVNKSKTHISVNGRKIRMRCRAIDGDVTFCRPKSPVYVGNGVHANLH
VAFHRSSSEKIHSNEISSDSIGVLGYQKTVDHTKVNSKLSLFFEIKSKTKEYAEKAKN
AYEKAKNAYQKANQAVLKAKEASSYDYILGWEFGGGVPEHKKEENMLSHLYVSSKDKENI
SKENDDVLDEKEEEAEETEEEELE[LEHHHHHH] (SEQ ID NO: 85).
**PP22 ;** sequence of MSP3-2 in italics-underlined (PlasmodB ID : PF3D7_1035500), residues 178 to 257 (79 aa) 
[MKYLLPTAAAGLLLLAAQPAMAMG]LNNNILGWEFGGGAPQNGAAEDKKTEYLLEQI
KIPSWDRNNIPDENEQVIEDPQEDNKDEDEDEETETENLETEDDNNEEGADDVVDSSKS
FVMENFSSYHGTKPGYVDSIQKGIQKPKSGTQGNYDDDWKEFYSTDNKYDAAG
YSVDNENPLSGKAGGVVKVTYPGLTKVLALKVDNAETIKKELGLSLTEPLMEQV
GTEEFIKRFGDGASRVVLSLPFAEGSSSVEYINNWEQAKALSVELEINFETRGKR
GQDAMYEYMAQACAGNRVRRSVGSSLSCINLDWDVIRDKTKTKIESLKEHGPIK
NKMSESPNKTVSEEKAKQYLEEFHQTALEHPELSELKTVTGTNPVFAGANYAAW
AVNVAQVIDSETADNLEKTTAALSILPGIGSVMGIADGAVHHNTEEIVAQSIALSSL
MVAQAIPLVGELVDIGFAAYNFVESIINLFQVVHNSYNRPAYSPGHKTQPFLHDGY
AVSWNTVEDSIIRTGFQGESGHDIKITAENTPLPIAGVLLPTIPGKLDVNKSKTHIS
VNGRKIRMRCRAIDGDVTFCRPKSPVYVGNGVHANLHVAFHRSSSEKIHSNEISS
DSIGVLGYQKTVDHTKVNSKLSLFFEIKSLNNNILGWEFGGGAPQNGAAEDKKTEY
LLEQIKIPSWDRNNIPDENEQVIEDPQEDNKDEDEDEETETENLETEDDNNEE[LEH
HHHHH] (SEQ ID NO: 86).
**PP23** ; sequence of MSP3-2 in italics-underlined before CRM197 (PlasmodB ID : PF3D7_1035500), residues 178 to 257 (79 aa) and MSP3-3 in italics after CRM197 (PlasmodB acc. Nbr PF3D7_1035600), residues 246 to 307 (61 aa). 
[MKYLLPTAAAGLLLLAAQPAMAMG]LNNNILGWEFGGGAPQNGAAEDKKTEYLLEQI
KIPSWDRNNIPDENEQVIEDPQEDNKDEDEDEETETENLETEDDNNEEGADDVVDSSKS
FVMENFSSYHGTKPGYVDSIQKGIQKPKSGTQGNYDDDWKEFYSTDNKYDAAG
YSVDNENPLSGKAGGVVKVTYPGLTKVLALKVDNAETIKKELGLSLTEPLMEQV
GTEEFIKRFGDGASRVVLSLPFAEGSSSVEYINNWEQAKALSVELEINFETRGKR
GQDAMYEYMAQACAGNRVRRSVGSSLSCINLDWDVIRDKTKTKIESLKEHGPIK
NKMSESPNKTVSEEKAKQYLEEFHQTALEHPELSELKTVTGTNPVFAGANYAAW
AVNVAQVIDSETADNLEKTTAALSILPGIGSVMGIADGAVHHNTEEIVAQSIALSSL
MVAQAIPLVGELVDIGFAAYNFVESIINLFQVVHNSYNRPAYSPGHKTQPFLHDGY
AVSWNTVEDSIIRTGFQGESGHDIKITAENTPLPIAGVLLPTIPGKLDVNKSKTHIS
VNGRKIRMRCRAIDGDVTFCRPKSPVYVGNGVHANLHVAFHRSSSEKIHSNEISS
DSIGVLGYQKTVDHTKVNSKLSLFFEIKSSNEKGRPPTYSPILDDGIEFSGGLYFNEKK
STEENKQKNVLESVNLTSWDKEDIVKENEDVK[LEHHHHHH] (SEQ ID NO: 87).
**PP25** ; sequence of LSA-3 in italics-underlined (PlasmodB ID : PF3D7_0220000), residues 176 to 325 (150 aa) 
[MKYLLPTAAAGLLLLAAQPAMAMG]SDELFNELLNSVDVNGEVKENILEESQVNDDIF
NSLVKSVQQEQQHNVEEKVEESVEENDEESVEENVEENVEENDDESVASSVEESIASSVDES
IDSSIEENVAPTVEEIVAPTVEEIVAPSVVESVAPSVEESVEENVEESVAENVEEGADDVVDS
SKSFVMENFSSYHGTKPGYVDSIQKGIQKPKSGTQGNYDDDWKEFYSTDNKYDA
AGYSVDNENPLSGKAGGVVKVTYPGLTKVLALKVDNAETIKKELGLSLTEPLME
QVGTEEFIKRFGDGASRVVLSLPFAEGSSSVEYINNWEQAKALSVELEINFETRG
KRGQDAMYEYMAQACAGNRVRRSVGSSLSCINLDWDVIRDKTKTKIESLKEHG
PIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSI
ALSSLMVAQAIPLVGELVDIGFAAYNFVESIINLFQVVHNSYNRPAYSPGHKTQPF
LHDGYAVSWNTVEDSIIRTGFQGESGHDIKITAENTPLPIAGVLLPTIPGKLDVNK
SKTHISVNGRKIRMRCRAIDGDVTFCRPKSPVYVGNGVHANLHVAFHRSSSEKIH
SNEISSDSIGVLGYQKTVDHTKVNSKLSLFFEIKSSDELFNELLNSVDVNGEVKENILE
ESQVNDDIFNSLVKSVQQEQQHNVEEKVEESVEENDEESVEENVEENVEENDDESVASSVE
ESIASSVDESIDSSIEENVAPTVEEIVAPTVEEIVAPSVVESVAPSVEESVEENVEESVAENVEE[
LEHHHHHH] (SEQ ID NO: 88).
LSA5-CRM Protein Sequence (SEQ ID NO: 89)

IPEEQIEEVIQEEIIEQVVPEELIEEVVPEEIIEEVIPEEIVEEVIYEEVIPEELVEEVIAEKLVKE
IVPEQVREEVTLEEIVEEMIPEEFVEEVAPEVEIEEIIPEELIEEVIPEVLVEEAVPEELIEKVIP
GADDVVDSSKSFVMENFSSYHGTKPGYVDSIQKGIQKPKSGTQGNYDDDWKEFYST
DNKYDAAGYSVDNENPLSGKAGGVVKVTYPGLTKVLALKVDNAETIKKELGLSLTE
PLMEQVGTEEFIKRFGDGASRVVLSLPFAEGSSSVEYINNWEQAKALSVELEINFETR
GKRGQDAMYEYMAQACAGNRVRRSVGSSLSCINLDWDVIRDKTKTKIESLKEHGPI
KNKMSESPNKTVSEEKAKQYLEEFHQTALEHPELSELKTVTGTNPVFAGANYAAWA
VNVAQVIDSETADNLEKTTAALSILPGIGSVMGIADGAVHHNTEEIVAQSIALSSLMV
AQAIPLVGELVDIGFAAYNFVESIINLFQVVHNSYNRPAYSPGHKTQPFLHDGYAVSW
NTVEDSIIRTGFQGESGHDIKITAENTPLPIAGVLLPTIPGKLDVNKSKTHISVNGRKIR
MRCRAIDGDVTFCRPKSPVYVGNGVHANLHVAFHRSSSEKIHSNEISSDSIGVLGYQK
TVDHTKVNSKLSLFFEIKSIPEEQIEEVIQEEIIEQVVPEELIEEVVPEEIIEEVIPEEIVEEVI
YEEVIPEELVEEVIAEKLVKEIVPEQVREEVTLEEIVEEMIPEEFVEEVAPEVEIEEIIPEELIE
EVIPEVLVEEAVPEELIEKVIPHHHHHH

**The Human Immunogenicicty Mouse Model (HIMM):** the model is based upon the engraftment of immunodeficient NOD-SCID-IL-2rynull (NSG) mice with human spleen lymphocytes (Hu-SPL-NSG), and complements the information obtained using ordinary laboratory mice such as Balb/C and C57BL.

### Immunization of Balb/c or C57Bl/6 mice and blood sampling

Groups of 4 to 5 Balb/c or C57Bl/6 mice received 2 or 3 injections of 10 µg of MSP3-1-LSP or 1 µg of PP21 diluted in 100 µl of Phosphate Buffer Saline pH 7.2 (PBS) and emulsified in 100 µl of adjuvant Montanide ISA 720. Immunizations were performed by subcutaneous injection and repeated once or twice fifteen days apart. Blood collection was performed by tail bleeding starting two weeks after the second injection and repeated at fifteen days interval up to 160 days afetr the first antigen injection. Sera were separated and stored at -20°C until used. Other groups handled simultaneously in parallel, received either 1 µg or 0.2 µg of PP21.

### Determination of anti-MSP3-LSP antibody titers in mice sera

MSP3-LSP specific antibody titers were determined in mice sera by Enzyme Linked Immunosorbent Assay (ELISA). Flat-bottomed microtitration plates (Nunc-Thermo Scientific, USA) were coated overnight at 4°C with 2µg/ml of PBS diluted MSP3-1-LSP or one of the MSP3-1-LSP peptides a, b, c or d,. After washing (PBS, pH 7.2) and saturation (PBS, 3% non-fat milk), serial dilutions of test sera (diluted in PBS, 3% non-fat milk, 0.05% Tween20) were added and incubated for 1 hour. Negative control consists of a preimmune mouse serum. Specific antibodies were revealed by subsequent addition of horseradish peroxidase-conjugated goat anti-mouse IgG (H+L) (Invitrogen, USA) for one hour, followed by the peroxidase substrate Tetramethylbenzidin (Amresco, USA). Specific antibody titer corresponds to the reverse of the last positive dilution.

### Human spleen donation and ethical considerations

The human spleens were obtained from deceased organ transplant donors according to an ethical agreement with the National Organization for Organ & Tissues Donation & Transplantation (NOOTDT) in Lebanon. The informed consent to donate organs for transplantation or scientific research was signed by the patients themselves during their lifetime or by their parents following the death.

### Human spleen cell preparation

Human spleens were processed within 24 hours after surgical excision as previously described (Brams P, 1998). Briefly, splenic tissue was dissected and a cell suspension was prepared. Red blood cells were lyzed using Gey's solution for 5-10 min at 25°C. After washing, leucocytes were resuspended in medium consisting of 37% fetal calf serum (FCS) (Sigma,USA), 10% dimethyl sulfoxide (DMSO) (Sigma,USA) and 53% RPMI 1640 (Sigma,USA), and then cryopreserved in liquid nitrogen until used. The mean number of cells isolated from each spleen donor was 10 ± 4 billion.

### Engraftment and immunization of human spleen cells in NSG mice

NOD.Cg-Prkdcscid-IL2rytmlWjI /SzJ (NSG) mice were obtained from The Jackson Laboratories (USA) and housed in sterile microisolators. All food, water, caging and bedding was autoclaved before use. Six- to 8-week old NSG mice were included in the experiments. Human spleen cells were cultured on day 0 at 4 × 10⁶ cells/ml with or without 1 µg/ml of antigen in RPMI1640 medium supplemented with 10% Fetal Calf Serum, 1% non-essential amino acids (NEAA 100x), 2 mM glutamine, 2 mM sodium pyruvate and 50 µg/ml gentamicin (complete medium). All culture reagents were purchased from Sigma, USA. On day 1, recombinant human IL-2 (Gibco Invitrogen) was added at 25 IU/ml. On day 3, the spleen cells were harvested, washed and resuspended in Hank's Balanced Salt Solution (HBSS). Each NSG mouse received an intraperitoneal injection (ip) of 30 × 10⁶ antigen-primed or unprimed spleen cells. At day 7, and day 21, reconstituted mice (Hu-SPL-NSG) were boosted with 10 µg of antigen injected intraperitoneally (ip) in 200 µl of HBSS-Montanide ISA 720 or MF59 adjuvant (v/v). Mice reconstituted with unprimed spleen cells, received only adjuvant. Blood samples were collected one week after each booster.

### Determination of human MSP3-1-LSP specific antibodies in Hu-SPL-NSG mice sera

These determinations were performed by ELISA. Briefly, for the detection of total human IgG in mouse serum, flat-bottomed microtitration plates (Nunc-Thermo Scientific, USA) were coated overnight at 4°C with 2.5µg/ml purified goat anti-human IgG (H + L) (Invitrogen, USA) in 0.1M carbonate buffer, pH 9.5. After washing (PBS, pH 7.2) and saturation (PBS, 3% non-fat milk), test sera (diluted in PBS, 3% non-fat milk, 0.05% Tween 20) were added and incubated for 1 hour. Negative controls consisted of preimmune mouse serum of the same animals. Human IgG were revealed by subsequent addition of horseradish peroxidase-conjugated goat anti-human IgG (H+L) (Invitrogen, USA) for one hour, followed by the peroxidase substrate Tetramethylbenzidin (Amresco, USA). Total human IgG concentration in the Hu-SPL-NSG mouse serum was calculated in comparison with a standard human IgG solution (Zymed, USA). The same test was performed for the detection of antigen specific antibodies, except that plates were coated with MSP3-1-LSP at 2.5µg/ml in PBS. In this case, negative controls consisted of sera of individuals that have never been infected with *Plasmodium,* while positive controls consisted of a pool of sera from hyperimmune African adults. In both cases, serial dilutions of each serum were tested in order to determine antibody titer. For specific antibody titer determination, a test was considered positive when it yielded an absorbance above cut-off (negative control OD x 2). The detection of IgG subclasses was performed using secondary mouse monoclonal antibodies specific for human IgG subclasses (clones NL16 [IgG1; Skybio, UK], HP6002 [IgG2; Sigma-Aldrich, Germany], and Zg4 and GB7B [IgG3 and IgG4, respectively; Skybio, UK) were used at final dilutions of 1/4,000, 1/10,000, 1/10,000, and 1/60,000, respectively), followed by horseradish peroxydase conjugated goat anti-mouse IgG diluted 1/4000 in PBS (Invitrogen).

### Quantification of gene expression of different cytokines, chemokines and transcription factors by Real-Time Reverse Transcription PCR (RTqPCR)

Spleen cells of immunized and non immunized mice were cultured at 2×10⁶ cells/ml in complete RPMI1640 medium, with or without stimulation with the immunizing antigen. Total cellular RNA was extracted after a 24h culture using RNeasy Mini Kit (Quiagen, Germany). Reverse transcription was carried out using RevertAid M-MuLV enzyme (RevertAid kit First Strand Synthesis kit, Thermo Scientific Fermentas) in a 20 µl reaction mixture. Briefly, 1 µl of oligo dT18 primer was added to about 400 ng of total RNA, mixed and incubated at 65°C for 5 min. The tube was placed on ice for a few minutes and centrifuged briefly before the addition of 5x reaction buffer, Ribolock Rnase inhibitor (20 u/ µl), dNTPs (10 mM) and reverse transcriptase (200 u/µl) as recommended by the manufacturer. The reaction mixture was incubated at 42°C for 60 min. The enzyme was inactivated by heating at 70°C for 5 min. Quantitative PCR was used to measure the relative expression of different human cytokines, chemokines and transcription factors considered as characteristic for Th1 or Treg cells. The PCR mixture was performed using LightCycler 480 SYBR Green Master, La Roche as recommended by the manufacturer. PCR amplifications were performed at 95°C for 5 min (1 cycle), followed by 45 cycles of incubation at 95°C for 10 sec and 55-57°C for 10 sec in the LightCycler 480 machine (La Roche). Each sample was run in triplicate. The housekeeping genes used as internal references were beta-actin (β-actin) and hypoxanthine phosphoribosyltransferase 1 (HPRT1) (geNorm analysis). The cycle threshold (Ct) value was calculated based on the produced PCR curve. The relative expression levels were calculated by the 2ΔΔCt method.

### Immunofluorescence assays.

Immunofluorescence assays were performed using air-dried, acetone-fixed, thin smears from red blood cell cultures containing predominantly mature schizonts of *P. falciparum* (3D7). Hu-SPL-NSG Sera diluted in PBS-1% bovine serum albumin were incubated at 37°C in a humid chamber for 1 h. After being washed with PBS, antibodies were detected by using an Alexa Fluor-conjugated goat anti-mouse IgG (Molecular Probes and Invitrogen) diluted to 1/400 in PBS with Evans blue counterstain (1/200).

### Results

### Example 1: Immunogenicity of PP21, a CRMP97-MS3 fusion construct

The immune responses induced by PP21 were assessed in the following animal models:
- BALB-C mice
- C57BL mice
- *Saimiri sciureus* monkeys
- The human immunogenicity mouse model (HIMM), where cells from human spleen tissue grafted in immunocompromised NSG mice are immunized in vivo and sera and cells from the immunized animals are used for immuno-assays.

All experiments were performed in comparative manner with the benchmark antigen MSP3-LSP (long synthetic peptide covering the 186-271 region of MSP3-1) used in clinical experiments, and using Montanide ISA 720 because laboratory mice respond usually poorly to alum, because the route of injection in HIMM does not allow to use alum, and above all because we were aiming at comparing constructs and not adjuvants, knowing that in humans alum adjuvated CRM (in meningococcal or pneumococcal vaccines) as MSP3 LSP provide very good responses.

Yet a succesful immunization with alum was also performed in lab mice, yielding titers of > 25 600 in Balb/C,even after two immunisarions, and 3200-25600 in C57bl (Figure 19).

### A major improvement of immunogenicity was observed using the PP21 Bio-fusion MSP3-1 construct of the invention, as compared to MSP3-LSP in mice.

PP21 induces higher serological responses of the target cytophilic subclasses than the benchmark antigen MSP3-LSP in all models used.
1. Low doses are very efficient: As with other CRM conjugated vaccines a major increase in immunogenicity was obtained, indicating that the fusion procedure produced as efficient products as chemical conjugation. We readily employed markedly lower dosages, which led to compare only 1 µg of PP21 with 10 µg of the reference MSP3-LSP (10 µG was found in the past not significantly different in lab mice from the 15 µG employed in the clinic). These differences were determined by different means see Figures 6 to 9. Results were reproducible with 4 distinct batches of PP21 in 4 groups of Balb/C and C57bl mice. Further dose-finding with PP21 focussed on lower dosage, eg 0.2 µg compared to 1 µg investigated in mice and in Saimiri monkeys. The high immunogenicity led us also to investigate only 2 as compared to 3 immunisations.
2. Wider breadth of recognition of ADCI targets : The peptides "b", "c" and "d" contained within the vaccine construct define distinct B-cell epitopes targeted by antibodies effective in ADCI. Reactivity with these peptides were higher in sera of mice vaccinated with PP21 as shown in Figure 9 (right-hand panel) as compared to the current MSP3-1-LSP (left-hand panel), at 1 and 10 µg respectively, suggesting that the magnitude and breadth of recognition of ADCI targets are higher and wider with PP21. In agreement with this finding the pattern of recognition of the 6 members of the MSP3 family, which exhibit cross-reactions through peptide « b » and « c-d » mainly, was wider with PP21. This also means that antibodies elicited by PP21 will trigger ADCI through triggering by antibody bridges to 6 proteins on the merozoite surface and not only one.
3. Very high B cell responses in Human lymphocytes : More importantly, human B-cell responses as assessed in the HIMM model showed similar major improvements with immunogenicity increased by 40-100 times. (Figure 10). In parallel experiments, using PP21 head to head with the present MSP3-LSP of 96 amino-acid, antibody titers were 20 to 100 fold higher with 1 µg of PP21 than with 10 µg of MSP3-LSP. Results were reproducible in 3 groups of 5 animals belonging to 3 HLA-classes.
4. Results are also reproducible when using either Montanide ISA720 or MF59 as adjuvants (Figure 14)
5. High IFN-γ secretion was obtained when Human lymphocytes were in vitro challenged with the parasite component MSP3-LSP (Figure 11). This important result indicates that CRM Th epitopes did not override the plasmodium-specific MSR3 T-cell epitopes and therefore that a Tcell help can be expected from vaccinated individuals when challenged by the parasite, yielding great chances for a fast anamnestic antibody rise when it is needed.
   Detailed analysis of cytokine profiles indicate that the responding cells are predominantly of a Thelper type 1 type (Figure 12 showing examples of results obtained with CXCL10 and Tbet)
6. Dominance of cytophilic IgG1: Isotype studies revealed as expected with strong Th1 responses a large dominance of the cytophilic isotypes, almost entirely made of IgG1 which is also the dominant isotype in MSP3-LSP immunized individuals. This was true when using either Montanide or MF59 as adjuvants (Figures 15 and 16). Although these result were obtained in a human model they bode very well of future responses which could be elicited in volunteers.
7. Reactivity with native parasite proteins: Antibodies elicited by PP21 vaccination reacted with the parasite native proteins in IFAT (Figure 13) and Western Blots The reactivity of antibodies, not only with the synthetic antigens, but above all with the parasite proteins is a critical feature to activate the ADCI defence mechanism during a malaria attack, and for the proper boosting of antibody responses by natural parasite challenges.
8. Very low immunizing doses are efficient: 0.2 µg and 1 µg per immunisation were assessed compared to 10 µg for MSP3-LSP (Figures 17) . At the one µg dose of PP21 a plateau was reached after the 2^{nd} immunisation, whereas at a 0.2 µg dose a third immunisation was required to reach about the same high levels of antibodies, and one animal did not respond. Yet the one microG profile indicates that 2 immunisations might be sufficient, and hence may deserve being explored.
9. Similar high immunogenicity is observed in South American Saimiri sciureus monkeys (Figure 20): 1 µg and 0.2 µg PP21 adjuvanted by Montanide 720 were used to immunize South American primates that respond usually poorly to immunisation. High responses close to titers seen in mice were reached at 1 µg, (see Figure 18) and about 5 fold lower titers at 0.2 µg (not shown). Though the comparison of titers in the same experiment has not yet be performed by our Brazilian colleagues, MSP3 LSP was very poorly immunogenic when it was employed in this model.
10. The duration of immune responses is markedly improved (Figure 8 upper and lower panels): Long term analysis showed a marked improvement of an essential component; the duration of antibody responses: Mice immunized by PP21 or LSP were followed up for 6 months post immunisation. The decay of titers is progressive but slow, so that at 6 months the titers remaining are higher than the peak obtained using MSP3-LSP. Titers remained with PP21 in the range 1/200 000 - 1/300 000 for more than 6 months, compared to a range of ca 1/50 000 for the current MSP3-LSP.

### Example 2: Immunogenicity of PP25, a CRMP97-LSA3 fusion construct

LSA3 is a pre-erythrocytic stage antigen, which has many attractive features, it was discovered by the differential responses of protected versus non-protected volunteers, it is conserved across strains, it is highly antigenic and immunogenic in a large range of animals including higher primates and chimpanzees, and induces in the latter immune responses protective against massive challenges by the human parasite Plasmodium falciparum at sporozoite stage. Protection is associated with antigen-specific Interferon gamma responses. The immunogenicity of PP25 was compared to control construction using LSA3 without conjugate nor any fusion (DG729). Conditions of immunization and immune-analysis are identical to those described above with PP21.

A similar major increase in antibody responses with LSA3-CRM97 biofusion, even using the antigen at low doses, was observed as was the case for MSP3 in PP21. The increase in IFN-γ responses was also seen but was less marked as LSA3 (DG729) without carrier is already a very good inducer of IFN-g responses.

The increase recorded in balb/C and C57bl mice, was also confirmed in the Human Immunogenicity model.

We further showed a very strong correlation between anti-LSA3 antibody titers with inhibition of P.falciparum parasites at sporozoite stage.

See results shown in **Figures 21-26****.**

### Example 3: Immunogenicity of PP23, a MSP3-2-CRMP97-MSP3-3 fusion constructs

The results obtained with PP21 relying on the fusion of MSP3-1 with CRM, and with LSA3, proved to be reproducible using other antigens from the erytrocytic stages such as MSP3-2 and MSP3-3 in a construct with CRM, named PP23. Indeed, strong B and T cell responses were elicited in Human Lymphocytes grafted in the HIMM model, by the construct PP23 (MSP3-2-CRMP97-MSP3-3) **(****Figure 27****),** the titers being on average 10 fold higher than in mice immunized by MSP3-2 CT alone in Montanide. Antibody sub-classes were predominantly of the IgG1 class, which is the main cytophilic class able to act in cooperation with monocytes in the ADCI mechanism. Similarly strong T-cells responses were induced as measured by secretion of IFN-γ by PP23-immunized human lymphocytes restimulated in vitro by either MSP3-2 or MSP3-3.

### Example 4 : LSA5-CRM

Liver-stage antigen-5 (LSA5), also known as PEBS (pre-erythrocytic and blood stages antigen), or SR 11.1 (sub-region of 11.1 P.falciparum gene) combines in a single molecule the features of both MSP3 and LSA3. It contains a unique sequence that differentiates it from the remaining of the mega-gene Pf 11.1, and is fully conserved across strains. It was recognized by sera from volunteers immunized with radiation- attenuated sporozoites and is unique as it induces protection against both pre-erythrocytic and asexual blood stages. It is expressed in both pre-erythrocytic and blood stages. The protective role of LSA5 against pre-erythrocytic stages is supported by convergent in vitro invasion inhibition studies, in vivo protection by passive transfer of anti-LSA5 antibodies, and by proof-of-concept studies in primates protected from challenge by vaccination with recombinant LSA5. Against the blood-stages, both naturally-occurring human and artificially-induced animal anti-Pf LSA5 antibodies exert a parasite-killing ADCI-mediated effect. Antigenicity is high in individuals from endemic areas with IgG3 antibodies predominating in individuals with premunition ie. exposure induced protection. A large number of epidemiological studies found strong association with protection against clinical malaria attacks and improved prognosis of drug-treated cerebral malaria. Finally LSA5 is highly immunogenic, achieving remarkably high titers in mice and greater ADCI activity than sera from MSP3-immunized mice.

A Bio-fusion of LSA5 with CRM197 similar in its principles to the previous 3 examples, was expressed in E.coli, its antigenicity ascertained and preliminary immunogenicity studies conducted. LSA5 immunogenicity was very high using the fusion with CRM (Figure 28); immunogenicity of 1 µg was compared to 10 µg dose of Bio-fusion LSA5-CRM/Montanide ISA720 in BALb/c mice (n=5). Experimental conditions were similar to those described in Figure 8 with MSP3. Immunization by Bio-fusion LSA5-CRM /Montanide ISA720 was performed on days 0,14 and 28 (arrows). Serum antibody detection was performed by ELISA titration on LSA5-LSP as antigen, using sera collected on days 0, 7, 21, 42.

## Claims

1. A biofusion malaria vaccine comprising an antigenic amino acid sequence of Plasmodium recognized by specific antibodies and/or lymphocytes, said sequence being fused with an immunogenic molecule from a microbial toxin, said biofusion malaria vaccine eliciting specific immune responses, and protective immunological responses against a Plasmodium infection *in vivo.*

2. The vaccine of claim 1, wherein the immunogenic molecule is selected from the group consisting of a cross-reacting material (CRM) of diphtheria toxoid, diphtheria toxoid (D), a non-toxic mutant recombinant Pseudomonas aeruginosa exoprotein A (repA), meningococcal outer membrane protein complex (OMPC), tetanus toxoid (T), *H. influenza* protein D (hiD), and immunogenic fragments thereof, in particular, wherein the immunogenic molecule is CRM197 or an immunogenic fragment thereof.

3. The vaccine of claim 2, wherein the immunogenic fragment is selected from the group consisting of Fragment A, Transmembrane Domain T, Receptor Binding domain R of CRM197, amino acid sequence 1-190 of CRM197, and amino acid sequence 1-389 of CRM197.

4. The vaccine according to any of claims 1 to 3, wherein the antigenic amino acid sequence is an antigenic protein which is expressed by Plasmodium at erythrocytic stage, or an epitopic fragment thereof.

5. The vaccine of claim 4, wherein the antigenic amino acid sequence is selected from the group consisting of MSP3, LSA5 (also named PEBS or sub-region of 11-1), Glurp R0 and R2, SERP, P27 and P27A, P45, P90 and P77, P14, P181, MSP1-Block 2, MSP2, GBP 130, protein 332, protein 11-1, MSP4, or any epitopic fragment thereof, in particular wherein the antigenic amino acid sequence is MSP3 or an epitopic fragment thereof.

6. The vaccine of claim 5, wherein the epitopic fragment encompasses motifs a, b, c, d, e and/or f of the C-terminal region of a Plasmodium MSP3 protein chosen among MSP3-1, MSP3-2, MSP3-3, MSP3-4, MSP3-7, and MSP3-8.

7. The vaccine of claim 6, wherein the Plasmodium MSP3 protein is MSP3-1.

8. The vaccine according to any of claims 1 to 3, comprising two antigenic amino acid sequences which are either each located in C-term and N-term of the immunogenic molecule, or both located at the same terminus.

9. The vaccine according to any of claims 1 to 3, wherein the antigenic amino acid sequence is linked at the N-term and/or C-term of the immunogenic molecule by a peptide linker, in particular wherein the peptide linker is a sequence of 1 to 35 amino acids, especially wherein the peptide linker is (Gly-Gly-Gly-Gly-Ser)ₙ, (Gly)ₙ or (EAAAK)n, wherein n is 1 to 4.

10. A nucleic acid construct encoding the biofusion as defined in any of claims 1 to 9.

11. A vaccine comprising the biofusion as defined in any of claims 1 to 7, or a combination of at least two biofusions as defined in any of claims 1 to 7, which respectively comprise at least two different antigenic amino acid sequences, or a nucleic acid construct encoding said biofusion(s), as defined in claim 10 with a physiologically acceptable vehicle.

12. The vaccine of claim 12, for use in vaccinating a human subject against malaria.

13. A vaccine comprising:
a. a fusion protein which comprises at least one antigenic amino acid sequence fused at the N-term and/or C-term of a carrier heterologous protein sequence, wherein the antigenic sequence comprises an epitope sequence of a Plasmodium protein and the carrier heterologous protein sequence is a sequence that is immunogenic in humans, wherein the carrier heterologous protein sequence is CRM197 or an immunogenic fragment thereof; which immunogenic fragment is selected from the group consisting of Fragment A, Transmembrane Domain T, Receptor Binding domain R of CRM197, amino acid sequence 1-190 of CRM197, and amino acid sequence 1-389 of CRM197; and wherein the antigenic sequence is MSP3, LSA3, or LSA5, or an epitopic fragment of MSP3 that encompasses motifs a, b, c, d, e and/or f of the C-terminal region of a Plasmodium MSP3 protein chosen among MSP3-1, MSP3-2, MSP3-3, MSP3-4, MSP3-7, MSP3-8, preferably selected from SEQ ID NO: 7 to SEQ ID NO: 54, or an epitopic fragment of LSA3 that is peptide DG729 of SEO ID NO: 77, or
b. a combination of at least two fusion proteins, where a fusion protein is as defined in a., which respectively comprise at least two different antigenic amino acid sequences, or
c. a nucleic acid construct encoding a fusion protein as defined in a.
with a physiologically acceptable vehicle.

14. The vaccine of claim 13, for use in vaccinating a human subject against malaria.

15. A method of preparation of a biofusion protein comprising:
(i) a step of construction of a nucleotidic sequence encoding an heterologous aminoacid sequence of an antigenic epitope of Plasmodium fused in N or C terminal, with a nucleotidic sequence encoding an amino-acid sequence of a carrier heterologous toxoid protein sequence immunogenic in Humans and
(ii) a step of expression of the nucleotidic sequence of (i) inserted in a vector comprising in an expression cassette the nucleic acid construct transfecting a host cell and
(iii) a step of separation of the expressed product obtained in step (ii) and collection of the fusion protein.
